(19) Europäisches Patentamt / European Patent Office / Office européen des brevets

(11) **EP 2 354 175 B1**

(12) # EUROPEAN PATENT SPECIFICATION

(45) Date of publication and mention of the grant of the patent:
**04.07.2018 Bulletin 2018/27**

(21) Application number: **09829171.9**

(22) Date of filing: **27.11.2009**

(51) Int Cl.:
*C08G 64/30* (2006.01) *C07D 493/04* (2006.01)
*C08G 64/02* (2006.01)

(86) International application number:
**PCT/JP2009/070057**

(87) International publication number:
**WO 2010/061926 (03.06.2010 Gazette 2010/22)**

(54) **METHOD FOR STORING DIHYDROXY COMPOUND TO BE USED AS STARTING MATERIAL FOR POLYCARBONATE**

VERFAHREN ZUR LAGERUNG EINER ALS AUSGANGSMATERIAL FÜR POLYCARBONAT ZU VERWENDENDEN DIHYDROXYVERBINDUNG

PROCÉDÉ DE STOCKAGE DE COMPOSÉ DIHYDROXY DEVANT ÊTRE UTILISÉ COMME PRODUIT DE DÉPART POUR LE POLYCARBONATE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **28.11.2008 JP 2008305723**
**28.11.2008 JP 2008305724**
**28.11.2008 JP 2008305725**
**28.11.2008 JP 2008305726**

(43) Date of publication of application:
**10.08.2011 Bulletin 2011/32**

(60) Divisional application:
**18174469.9**

(73) Proprietor: **Mitsubishi Chemical Corporation**
**Tokyo 100-8251 (JP)**

(72) Inventors:
- **FUKUMOTO Takahiro**
  **Kitakyushu-shi**
  **Fukuoka 806-0004 (JP)**
- **KAGEYAMA Naoya**
  **Kitakyushu-shi**
  **Fukuoka 806-0004 (JP)**
- **NAMIKI Shingo**
  **Kitakyushu-shi**
  **Fukuoka 806-0004 (JP)**
- **FUJI Michiaki**
  **Yokkaichi-shi**
  **Mie 510-8530 (JP)**
- **YAMAMOTO Masanori**
  **Kitakyushu-shi**
  **Fukuoka 806-0004 (JP)**
- **NISHIDA Yuuichi**
  **Kitakyushu-shi**
  **Fukuoka 806-0004 (JP)**

(74) Representative: **Vossius & Partner**
**Patentanwälte Rechtsanwälte mbB**
**Siebertstrasse 3**
**81675 München (DE)**

(56) References cited:
**EP-A1- 1 243 607** **EP-A1- 2 033 981**
**WO-A1-02/06374** **WO-A1-02/22708**
**WO-A1-99/55764** **WO-A1-2004/111106**
**WO-A1-2009/075304** **JP-A- 6 032 885**
**JP-A- 2003 335 852** **JP-A- 2004 067 990**
**JP-A- 2006 028 441** **JP-A- 2008 024 919**
**JP-A- 2008 056 844** **JP-T- 2002 534 486**
**JP-T- 2005 509 667** **US-A1- 2003 097 028**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 354 175 B1

## Description

### TECHNICAL FIELD

The present invention relates to a method of storing a dihydroxy compound for use in a polycarbonate raw material, a method of preparing a polycarbonate raw material, and a method of producing a polycarbonate. More particular, the invention relates to a method of storage which inhibits a dihydroxy compound from deteriorating in quality during long-term storage and is effective in preventing the compound from compacting under load, a method of preparing a polycarbonate raw material which includes a dihydroxy compound and is used for stably producing a polycarbonate excellent in transparency, color tone, heat resistance, moldability, and mechanical strength and having excellent optical properties, and a method of producing a polycarbonate.

### BACKGROUND ART

Polycarbonates, which are generally produced from bisphenol compounds as monomer ingredients, are in extensive use as so-called engineering plastics in the fields of electrical and/or electronic parts and automotive parts and in the optical field including optical recording media and lenses so as to take advantage of the superiority thereof in transparency, heat resistance, mechanical strength, etc. However, for use as optical compensation films in flat-panel displays and the like, which are rapidly spreading recently, a higher degree of optical properties such as, e.g., low birefringence and a low photoelastic coefficient have come to be required. The existing aromatic polycarbonates have become unable to meet the requirement.

Conventional polycarbonates are produced from raw materials induced from oil resources. In recent years, however, there is a fear about the depletion of oil resources and it is desired to provide a polycarbonate produced from raw materials obtained from biomass resources such as plants. There also is a fear that the global warming effect of increasing carbon dioxide emission and accumulation thereof may bring about climate changes, etc. There is hence a desire for the development of a polycarbonate which is produced from plant-derived monomers and which, even when discarded after use, is carbon-neutral.

Under these circumstances, methods have been proposed in which a special dihydroxy compound is used as a monomer ingredient to obtain a polycarbonate through the transesterification of the dihydroxy compound with a carbonic acid diester (see, for example, patent documents 1 to 7).

Such dihydroxy compounds having a special structure generally have insufficient stability and are apt to deteriorate in quality through reaction with water or oxygen. However, none of patent documents 1 to 7 specifically describes long-term stability.

On the other hand, patent document 8 discloses that isosorbide, which is a typical dihydroxy compound, was evaluated for long-term stability by placing 20 g of isosorbide in a 50-mL glass container and storing this isosorbide at 40°C for 1 month in the presence of 3% water vapor.

Incidentally, in the case of producing a polycarbonate on an industrial scale, it is necessary to use the dihydroxy compound, as a raw material for the polycarbonate, in a large amount. Because of this, the dihydroxy compound in the state of being packed in a container is stored in a stock room or transported with a motor truck, ship, or the like in shipping. However, since such dihydroxy compounds often have a low melting point and are apt to deliquesce, the dihydroxy compounds during storage or transportation are apt to be compacted by the load of the raw material itself and become a fusion-bonded mass.

Once the dihydroxy compound becomes massive, this compound not only is difficult to handle especially in large-scale production but also is causative of various apparatus troubles.

Furthermore, in the case where a polycarbonate is to be obtained by the transesterification method, it is important to maintain a polymerization rate and to regulate the concentration of terminal groups, which influences quality, to a given value. For attaining this, it is necessary to precisely regulate a molar ratio between the monomers, i.e., the raw-material dihydroxy compound and a carbonic acid diester. However, when the dihydroxy compound which has become a solid mass or fusion-bonded mass is used as a raw material without any treatment, this compound cannot be precisely weighed, resulting in a problem that the polycarbonate obtained has unstable quality. There also has been a problem that the dihydroxy compound which has become massive often causes the generation of a substance that inhibits polymerization, resulting in a decrease in polymerization rate.

Consequently, in the case where the dihydroxy compound as a raw material for a polycarbonate is stored on an industrial scale, it is necessary to not only maintain stability to moisture, temperature, and (oxygen concentration) but also inhibit the dihydroxy compound from being compacted by the load of the raw material itself over a prolonged time period. However, none of those documents discloses any method of long-term storage for long-distance transportation with a ship or the like or in a stock room. In particular, no investigation has been made therein on the stability of the dihydroxy compound placed under load.

In general, the dihydroxy compounds having a special structure mostly have a high melting point, and are inferior in thermal stability to bisphenol compounds. Furthermore, those dihydroxy compounds often have a low rate of dissolution in carbonic acid diesters and other substances. Consequently, in the method in which raw materials are fed en bloc as described in known documents, the dihydroxy compound ingredient remains undissolved even after the carbonic acid diester has melted, and it is necessary to subject the mixture to an additional heat history in which the temperature is further elevated or a prolonged time period is used. However, such a heat history causes, through an undesirable side reaction, the generation of a substance inhibiting polymerization or imparts an impaired color tone and reduced thermal stability to the polycarbonate to be finally obtained. In addition, dihydroxy compounds having such a structure are apt to be deteriorated in quality by the presence of a slight amount of oxygen. However, when the raw material is used in the original solid state, it is difficult to completely remove the oxygen which has come into the interstices among the solid particles. Even if the solid particles are subjected repeatedly to vacuum degassing and replacement with, e.g., an inert gas, it is difficult to completely remove the oxygen. Furthermore, in the case where a polycarbonate is to be obtained by the transesterification method, it is important to maintain a polymerization rate and to regulate the concentration of terminal groups, which influences quality, to a given value. Although it is necessary to precisely regulate a molar ratio between the monomers, i.e., the raw-material dihydroxy compound and a carbonic acid diester, for attaining the desired control of polymerization, it is difficult in large-scale production to precisely weigh or meter the whole monomers to be fed as described in known documents. As a result, the resultant polycarbonate has impaired quality or unstable quality. Moreover, in the case where such a polycarbonate is produced on an industrial scale, the use of a solid monomer is apt to arouse troubles such as clogging or dust explosion. It is therefore desired to minimize unit operations involving a solid monomer.

Patent document 9 discloses a high-purity anhydrous sugar alcohol obtained by distilling and recrystallizing a dihydroxy compound and a process for producing the sugar alcohol.

On the other hand, patent document 10 describes a method in which a dihydroxy compound that is a product of internal dehydration of a hydrogenated sugar is purified by distillation and then brought into contact with a stability improver in order to improve the stability of the dihydroxy compound. Patent document 11 describes a polycarbonate produced from a dihydroxy compound having a specific structure and an alicyclic dihydroxy compound as raw materials and combining high heat resistance and transparency. This document includes a statement concerning a problem to the effect that decomposition products including formic acid generate through oxidation when the dihydroxy compounds are handled during storage or production thereof and these decomposition products cause the resultant polycarbonate to suffer coloration or property deterioration.

PRIOR-ART DOCUMENTS

PATENT DOCUMENTS

Patent Document 1: JP-A-2004-67990
Patent Document 2: International Publication 2004/111106
Patent Document 3: International Publication 2006/41190
Patent Document 4: JP-A-2006-232897
Patent Document 5: JP-A-2006-28441
Patent Document 6: JP-A-2008-56844
Patent Document 7: JP-A-2008-24919
Patent Document 8: JP-T-2005-509667
Patent Document 9: JP-T-2002-534486
Patent Document 10: JP-T-2005-509667
Patent Document 11: JP-A-2008-24919


SUMMARY OF THE INVENTION

PROBLEMS THAT THE INVENTION IS TO SOLVE

An object of the invention is to provide, under the circumstances described above, a method of storing the dihydroxy compound for use in a polycarbonate raw material, the method being effective not only in inhibiting the dihydroxy compound to be used in a raw material for a polycarbonate from deteriorating in quality even when stored for long, but also in preventing or inhibiting the dihydroxy compound from being compacted into a fusion-bonded mass during long-term storage, whereby the dihydroxy compound can retain a powder or pellet state.

Another object of the invention is to eliminate the conventional problems described above and eliminate the problems in polycarbonate production processes which are attributable to difficulty in evenly mixing a dihydroxy compound with a carbonic acid diester and to thereby provide: a method of preparing a raw material for easily and stably producing a polycarbonate excellent in transparency, color tone, heat resistance, moldability, and mechanical strength and having excellent optical properties; and a method of producing the polycarbonate.

MEANS FOR SOLVING THE PROBLEMS

The present inventors diligently made investigations in order to overcome the problems described above. As a result, the following findings were obtained.

(a) By regulating a specific solid-state dihydroxy compound with respect to the amount of water contained in the dihydroxy compound, the temperature of the container, and the pressure applied to the dihydroxy compound, the problems concerning quality deterioration and compaction are overcome and the dihydroxy compound can be stably stored over long.
(b) When a dihydroxy compound which has a specific structure in the molecule and is solid at 25°C is mixed with a molten carbonic acid diester at a specific pressure for a certain time period and the resultant mixture is used as a polycarbonate raw material, then a polycarbonate excellent in transparency, color tone, heat resistance, moldability, and mechanical strength and having excellent optical properties can be stably produced.
(c) When a dihydroxy compound ingredient including a dihydroxy compound having a specific structure in the molecule is mixed with a carbonic acid diester at a specific temperature and a specific pressure for a certain time period, then the dihydroxy compound evenly mixes with the carbonic acid diester. By adding an aliphatic and/or an alicyclic dihydroxy compound to the dihydroxy compound and carbonic acid diester, the mixing of the dihydroxy compound with the carbonic acid diester is further accelerated and a polycarbonate having especially high transparency can be produced.
(d) One of the causes of coloration of dihydroxy compounds and of polycarbonate products obtained therefrom is metal atoms contained as an impurity in the dihydroxy compounds. When a dihydroxy compound having a specific structure and having a metal atom content lower than 1 ppm is used as a polycarbonate raw material to be subjected to polymerization reaction, a polycarbonate having high transparency can be produced. Furthermore, by reducing the amount of aldehyde compounds contained in the dihydroxy compound, a polycarbonate having especially high transparency can be produced.

It was found that the following inventions meet those objects, and the present invention has been thus achieved.
Namely, the present invention relates to the following inventions.

(1) A method of storing a dihydroxy compound for use in a polycarbonate raw material which comprises packing a solid-state dihydroxy compound having the structure represented by the following formula (1) in the molecule into a container and storing the compound packed in the container, characterized in that the dihydroxy compound is stored under such conditions that the dihydroxy compound has a water content of 1.0% by weight or lower, the container has an internal temperature of 60°C or lower, and a pressure of from 0.005 kgf/cm$^2$ to 0.5 kgf/cm$^2$ is applied to the bottom of the container,
[Chem. 1]

$$-CH_2-O- \qquad (1)$$

(provided that the structure of formula (1) in which the oxygen atom is bonded to a hydrogen atom is excluded).
(2) The method of storage according to (1) preferably characterized in that the dihydroxy compound has a heterocyclic structure.
(3) The method of storage according to (1) or (2) preferably characterized in that the dihydroxy compound is represented by the following general formula (2).

[Chem. 2]

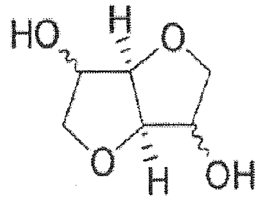

(2)

(4) The method of storage according to any one of (1) to (3) preferably characterized in that the atmosphere in the container has an oxygen concentration of from 0.0001 vol% to 10 vol%.

(5) The method of storage according to any one of (1) to (4) preferably characterized in that the container is a metallic container, a resinous container, a fiber drum, a flexible container, or a paper bag.

(6) The method of storage according to (5) preferably characterized in that the container is a container having an inner bag comprising a resinous film.

(7) The method of storage according to (6) preferably characterized in that the resinous film is a resinous film on which an inorganic layer having gas barrier properties has been formed.

(8) The method of storage according to any one of (1) to (7) preferably characterized in that the solid-state dihydroxy compound and a free-oxygen absorber are caused to coexist in the container while keeping the dihydroxy compound and the absorber not in contact with each other.

(9) The method of storage according to any one of (1) to (8) preferably characterized in that the dihydroxy compound has an average bulk density of from 200 $kg/m^3$ to 1,000 $kg/m^3$.

(10) The method of storage according to any one of (1) to (9) preferably characterized in that the dihydroxy compound has a formic acid content of 20 ppm by weight or lower.

Here also described are the following embodiments not falling under the present claims:

(11) A dihydroxy compound for use in a polycarbonate raw material, characterized by being a disaggregation product obtained by disaggregating the dihydroxy compound which has been stored by the method of storage according to any one of (1) to (10), the disaggregation product containing, per kg, 20 or less solid masses having a maximum length of from 3 cm to 30 cm.

(12) A method of preparing a polycarbonate raw material, characterized by mixing a dihydroxy compound which has the structure represented by the following formula (1) in the molecule and is solid at 25°C with a molten carbonic acid diester under the conditions of a pressure of from 0.06 MPa to 0.20 MPa and a period of from 0.5 hours to 30 hours and using the resultant mixture as a raw material for a polycarbonate,

[Chem. 3]

$$-CH_2-O- \qquad (1)$$

(provided that the structure of formula (1) in which the oxygen atom is bonded to a hydrogen atom is excluded).

(13) The method of preparing a polycarbonate raw material according to (12) preferably characterized in that the dihydroxy compound is brought into a melt, solution, or suspension state and then mixed with the molten carbonic acid diester and the resultant mixture is used as a raw material for a polycarbonate.

(14) The method of preparing a polycarbonate raw material according to (13) preferably characterized in that the dihydroxy compound is melted, dissolved, or suspended in an atmosphere having an oxygen concentration of from 0.0001 vol% to 10 vol%.

(15) The method of preparing a polycarbonate raw material according to any one of (12) to (14) preferably characterized in that the dihydroxy compound is brought into a melt or solution state, subsequently purified by distillation, and then mixed with the molten carbonic acid diester and the resultant mixture is used as a raw material for a polycarbonate.

(16) The method of preparing a polycarbonate raw material according to (15) preferably characterized in that the dihydroxy compound which has not been purified by distillation contains a basic stabilizer.

(17) The method of preparing a polycarbonate raw material according to any one of (12) to (16) preferably characterized in that the dihydroxy compound has a melting point of 220°C or lower.

(18) The method of preparing a polycarbonate raw material according to any one of (12) to (17) preferably characterized in that the operation of mixing the dihydroxy compound with the molten carbonic acid diester is conducted in an atmosphere having an oxygen concentration of from 0.0001 vol% to 10 vol%.

(19) The method of preparing a polycarbonate raw material according to any one of (12) to (18) preferably characterized by satisfying $B \leq 6A$, wherein A is the weight of the carbonic acid diester melt in terms of parts by weight and

B is the rate of feeding the dihydroxy compound in terms of parts by weight per hour.

(20) The method of preparing a polycarbonate raw material according to any one of (13) to (19) preferably characterized in that the melt of the dihydroxy compound is obtained by feeding the dihydroxy compound in a solid state to a melt obtained beforehand by melting the dihydroxy compound and melting the solid-state dihydroxy compound.

(21) The method of preparing a polycarbonate raw material according to (20) preferably characterized by satisfying D≤6C, wherein C is the weight in terms of parts by weight of the melt obtained by melting the dihydroxy compound and D is the rate of feeding the solid-state dihydroxy compound in terms of parts by weight per hour.

(22) The method of preparing a polycarbonate raw material according to any one of (12) to (21) preferably characterized in that the dihydroxy compound in a solid state has an average bulk density of from 200 $kg/m^3$ to 1,000 $kg/m^3$.

(23) The method of preparing a polycarbonate raw material according to any one of (12) to (22) preferably characterized in that the dihydroxy compound in a solid state is received with a receiver having a bottom cone angle of 120 degrees or smaller.

(24) The method of preparing a polycarbonate raw material according to any one of (12) to (23) preferably characterized in that the dihydroxy compound has a formic acid content of 20 ppm by weight or lower when mixed with the carbonic acid diester.

(25) The method of preparing a polycarbonate raw material according to any one of (12) to (24) preferably characterized in that the dihydroxy compound is represented by the following general formula (2).

[Chem. 4]

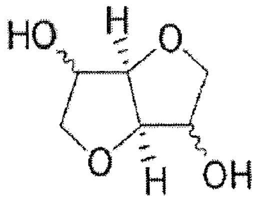

(2)

(26) The method of preparing a polycarbonate raw material according to any one of (12) to (25) preferably characterized in that the mixing of the dihydroxy compound with the carbonic acid diester is conducted at a temperature of from 70°C to 240°C.

(27) A method of producing a polycarbonate from a dihydroxy compound ingredient comprising at least a dihydroxy compound having the structure represented by the following formula (1) in the molecule and from a carbonic acid diester, characterized by mixing the dihydroxy compound having the structure represented by the following formula (1) in the molecule with the carbonic acid diester in a molten state at a temperature of from 70°C to 240°C and a pressure of from 0.06 MPa to 0.20 MPa for a period of from 0.5 hours to 30 hours and then causing a transesterification reaction to proceed at a pressure lower than 0.06 MPa to produce a polycarbonate,
[Chem. 5]

$$-CH_2-O- \qquad (1)$$

(provided that the structure of formula (1) in which the oxygen atom is bonded to a hydrogen atom is excluded).

(28) A method of producing a polycarbonate from a dihydroxy compound ingredient comprising at least a dihydroxy compound having the structure represented by the following formula (1) in the molecule and from a carbonic acid diester, characterized by mixing the dihydroxy compound having the structure represented by the following formula (1) in the molecule with the carbonic acid diester in a molten state at a temperature of from 70°C to 240°C and a pressure of from 0.06 MPa to 0.20 MPa for a period of from 0.5 hours to 30 hours, subsequently continuously feeding the resultant mixture to a reaction tank kept at a pressure lower than 0.06 MPa, and causing a transesterification reaction to proceed to produce a polycarbonate,
[Chem. 6]

$$-CH_2-O- \qquad (1)$$

(provided that the structure of formula (1) in which the oxygen atom is bonded to a hydrogen atom is excluded).

(29) The method of producing a polycarbonate according to (27) or (28) preferably characterized in that the dihydroxy compound having the structure represented by formula (1) in the molecule is mixed with the molten-state carbonic acid diester in a tank equipped with a stirrer, the stirrer having a stirring blade tip speed of 0.05-10 m/s.

(30) The method of producing a polycarbonate according to any one of (27) to (29) preferably characterized in that

the raw materials for use in the reaction are prepared so that the amount of the carbonic acid ester is 0.90-1.20 mol per mol of the dihydroxy compound ingredient.

(31) The method of producing a polycarbonate according to any one of (27) to (30) preferably characterized in that at least the dihydroxy compound having the structure represented by formula (1) in the molecule and an aliphatic and/or an alicyclic dihydroxy compound are used as the dihydroxy compound ingredient, and that the proportion of the aliphatic and/or the alicyclic dihydroxy compound is 0.01 mol or more per mol of the carbonic acid diester.

(32) The method of producing a polycarbonate according to (31) preferably characterized in that the aliphatic dihydroxy compound is 1,3-propanediol.

(33) The method of producing a polycarbonate according to (31) preferably characterized in that the alicyclic dihydroxy compound is 1,4-cyclohexanedimethanol and/or tricyclodecanedimethanol.

(34) The method of producing a polycarbonate according to any one of (27) to (33) preferably characterized in that the dihydroxy compound having the structure represented by formula (1) in the molecule is a heterocyclic dihydroxy compound.

(35) The method of producing a polycarbonate according to (34) preferably characterized in that the heterocyclic dihydroxy compound is represented by the following general formula (2).

[Chem. 7]

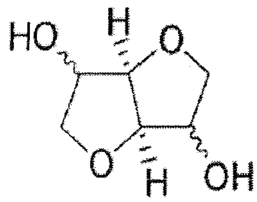

(2)

(36) The method of producing a polycarbonate according to any one of (27) to (33) preferably characterized in that the dihydroxy compound ingredient having the structure represented by formula (1) in the molecule is 9,9-bis(4-(2-hydroxyethoxy)phenyl)-fluorene.

(37) The method of producing a polycarbonate according to any one of (27) to (36) preferably characterized in that the dihydroxy compound ingredient is mixed with the molten carbonic acid diester at 80-120°C.

(38) The method of producing a polycarbonate according to (27) or (28) wherein the dihydroxy compound having the structure represented by formula (1) in the molecule is reacted with a carbonic acid diester to produce a polycarbonate, the method being preferably characterized in that the dihydroxy compound has a metal atom content lower than 1 ppm.

(39) The method of producing a polycarbonate according to (38) preferably characterized in that the metal atom content of the dihydroxy compound is lower than 0.5 ppm.

(40) The method of producing a polycarbonate according to (38) or (39) preferably characterized in that the dihydroxy compound has an aldehyde compound content lower than 0.01% by weight.

(41) The method of producing a polycarbonate according to (14) preferably characterized in that the aldehyde compound content is lower than 0.008% by weight.

(42) The method of producing a polycarbonate according to any one of (38) to (41) preferably characterized in that the dihydroxy compound having the structure represented by formula (1) in the molecule is distilled before being reacted.

(43) The method of producing a polycarbonate according to (42) preferably characterized in that the distillation is conducted so as to result in at least 2% by weight initial distillate and at least 8% by weight bottoms (provided that the amount of the dihydroxy compound subjected to the distillation is 100% by weight).

(44) The method of producing a polycarbonate according to (42) or (43) preferably characterized in that the distillation of the dihydroxy compound having the structure represented by formula (1) in the molecule is conducted with a distillation apparatus in which the part coming into contact with the dihydroxy compound comprises a molybdenum-containing alloy.

(45) The method of producing a polycarbonate according to any one of (38) to (44) preferably characterized in that the reaction between the dihydroxy compound and the carbonic acid diester is conducted in a reaction vessel which comprises a molybdenum-containing alloy.

(46) The method of producing a polycarbonate according to any one of (27) to (45) preferably characterized in that the disaggregated dihydroxy compound according to (11) is used as a raw material for the polycarbonate.

(47) The method of producing a polycarbonate according to any one of (27) to (45) preferably characterized in that a raw material prepared by the method according to any one of (1) to (15) is used.

ADVANTAGES OF THE INVENTION

According to the method of storage of the invention, a dihydroxy compound of formula (1) can be stored over long while inhibiting the compound from deteriorating in quality and preventing the compound from compacting.

BRIEF DESCRIPTION OF THE DRAWINGS

[Fig. 1] Fig. 1 shows one example of stirring tanks for melting a dihydroxy compound for use in a polycarbonate raw material according to the invention.
[Fig. 2] Fig. 2 is a diagrammatic view illustrating one embodiment of the method of preparing a polycarbonate of the invention.
[Fig. 3] Fig. 3 is a diagrammatic view illustrating one embodiment (stirring/mixing) of the method of producing a polycarbonate of the invention.
[Fig. 4] Fig. 4 is a diagrammatic view illustrating one embodiment (polymerization reaction) of the method of producing a polycarbonate of the invention.

MODES FOR CARRYING OUT THE INVENTION

Embodiments of the invention are explained below in the order of: (A) the method of storing a dihydroxy compound for use in a polycarbonate raw material.

(A) Method of Storing Dihydroxy Compound for Use in Polycarbonate Raw Material

The method of storing a dihydroxy compound for use in a polycarbonate raw material of the invention (hereinafter sometimes referred to simply as "method of storage of the invention") relates to a method of storing a dihydroxy compound which comprises packing a solid-state dihydroxy compound having the structure represented by the following formula (1) in the molecule (hereinafter often referred to as "solid dihydroxy compound according to the invention") into a container and storing the compound packed in the container, wherein the dihydroxy compound is stored under such conditions that the dihydroxy compound has a water content of 1.0% by weight or lower, the container has an internal temperature of 60°C or lower, and a pressure of from 0.005 kgf/cm$^2$ to 0.5 kgf/cm$^2$ is applied to the bottom of the container,
[Chem. 8]

$$-CH_2-O- \qquad (1)$$

(provided that the structure of formula (1) in which the oxygen atom is bonded to a hydrogen atom is excluded).

In the method of storage of the invention, the term "storage" means a conception which includes the state of the solid dihydroxy compound according to the invention which is at any point of time in the period from the time when the solid dihydroxy compound is packed into a container to the time when this compound is subjected to a specific application. Namely, the conception of the term "storage" as used in the invention includes not only the case where the solid dihydroxy compound according to the invention which in the state of being packed in a container is stored in a stock room but also the case where the compound in the packed state is being transported with a truck and/or a ship or the like.

With respect to the term "solid-state dihydroxy compound", the dihydroxy compound which is being stored is regarded as in a solid state, regardless of the melting point of the compound, so long as the compound is in a solid state even temporarily during "storage". Namely, even when the dihydroxy compound has a melting point not higher than ordinary temperature, the method is a method of storing a solid-state dihydroxy compound packed in a container, so long as the compound is stored at a temperature not higher than the melting point thereof. In the invention, the dihydroxy compound is referred to as a solid dihydroxy compound according to the invention.

Furthermore, in the method of storage of the invention, the term "pressure applied to the bottom of the container" means the pressure applied to the container bottom by the load of the solid dihydroxy compound itself according to the invention, etc. In the case of containers which are packed with the dihydroxy compound and then stacked, such as flexible containers or paper bags, that term means the pressure applied to the bottom of the lowest containers because the load of the upper packages directly influences.

Examples of the solid dihydroxy compound according to the invention include compounds having a side chain including an aromatic ring and further having an aromatic-group-bonded ether group in the main chain, such as 9,9-bis(4-(2-hydroxyethoxy)phenyl)fluorene, 9,9-bis(4-(2-hydroxyethoxy)-3-methylphenyl)fluorene, 9,9-bis(4-(2-hydroxyethoxy)-3-isopropylphenyl)fluorene, 9,9-bis(4-(2-hydroxyethoxy)-3-isobutylphenyl)fluorene, 9,9-bis(4-(2-hydroxyethoxy)-3-tert-butylphenyl)fluorene, 9,9-bis(4-(2-hydroxyethoxy)-3-cyclohexylphenyl)fluorene, 9,9-bis(4-(2-hydroxyethoxy)-3-phenylphenyl)fluorene, 9,9-bis(4-(2-hydroxyethoxy)-3,5-dimethylphenyl)-fluorene, 9,9-bis(4-(2-hydroxyethoxy)-3-tert-butyl-6-methylphenyl)fluorene, and 9,9-bis(4-(3-hydroxy-2,2-dimethylpropoxy)phenyl)fluorene, oxyalkylene glycols such as diethylene glycol, triethylene glycol, and tetraethylene glycol, bis(hydroxyalkoxy-aryl)alkanes such as bis[4-(2-hydroxyethoxy)phenyl]methane, bis[4-(2-hydroxy-ethoxy)phenyl]diphenylmethane, 1,1 -bis [4-(2-hydroxyethoxy)phenyl] ethane, 1,1-bis[4-(2-hydroxyethoxy)phenyl]-1-phenylethane, 2,2-bis[4-(2-hydroxyethoxy)phenyl]-propane, 2,2-bis[4-(2-hydroxyethoxy)-3-methylphenyl]propane, 2,2-bis[3,5-dimethyl-4-(2-hydroxyethoxy)phenyl]propane, 1,1-bis[4-(2-hydroxyethoxy)phenyl]-3,3,5-trimethylcyclohexane, 1,1-bis[4-(2-hydroxyethoxy)phenyl]cyclohexane, 1,4-bis[4-(2-hydroxyethoxy)phenyl]cyclohexane, 1,3-bis[4-(2-hydroxyethoxy)phenyl]cyclohexane, 2,2-bis[4-(2-hydroxyethoxy)-3-phenylphenyl]propane, 2,2-bis[(2-hydroxyethoxy)-3-isopropylphenyl]propane, 2,2-bis[3-tert-butyl-4-(2-hydroxyethoxy)phenyl]propane, 2,2-bis[4-(2-hydroxyethoxy)phenyl]butane, 2,2-bis[4-(2-hydroxyethoxy)phenyl]-4-methyl-pentane, 2,2-bis[4-(2-hydroxyethoxy)phenyl]octane, 1,1-bis[4-(2-hydroxyethoxy)-phenyl]decane, 2,2-bis[3-bromo-4-(2-hydroxyethoxy)phenyl]propane, and 2,2-bis[3-cyclohexyl-4-(2-hydroxyethoxy)phenyl]propane, bis(hydroxyalkoxyaryl)cycloalkanes such as 1,1-bis[4-(2-hydroxyethoxy)phenyl]cyclohexane, 1,1-bis[3-cyclohexyl-4-(2-hydroxyethoxy)phenyl]cyclohexane, and 1,1-bis[4-(2-hydroxyethoxy)phenyl]-cyclopentane, dihydroxyalkoxydiaryl ethers such as 4,4'-bis(2-hydroxyethyl)diphenyl ether and 4,4'-bis(2-hydroxyethoxy)-3,3'-dimethyldiphenyl ether, bishydroxyalkoxyaryl sulfides such as 4,4'-bis(2-hydroxyethoxyphenyl) sulfide and 4,4'-bis[4-(2-dihydroxyethoxy)-3-methylphenyl] sulfide, bishydroxyalkoxyaryl sulfoxides such as 4,4'-bis(2-hydroxyethoxyphenyl) sulfoxide and 4,4'-bis[4-(2-dihydroxyethoxy)-3-methylphenyl] sulfoxide, bishydroxyalkoxyaryl sulfones such as 4,4'-bis(2-hydroxyethoxyphenyl) sulfone and 4,4'-bis[4-(2-dihydroxyethoxy)-3-methylphenyl] sulfone, 1,4-bishydroxyethoxybenzene, 1,3-bishydroxyethoxybenzene, 1,2-bishydroxyethoxybenzene, 1,3-bis[2-[4-(2-hydroxyethoxy)phenyl]propyl]benzene, 1,4-bis [2- [4-(2-hydroxyethoxy)phenyl]propyl] benzene, 4,4' -bis(2-hydroxyethoxy)biphenyl, 1,3-bis[4-(2-hydroxyethoxy)phenyl]-5,7-dimethyladamantane, anhydrous sugar alcohols represented by a dihydroxy compound represented by the following formula (2), and compounds having a cyclic ether structure, such as the spiro-glycol represented by the following general formula (3). These compounds may be used alone or in combination of two or more thereof.

[Chem. 9]

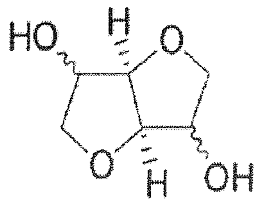

(2)

[Chem. 10]

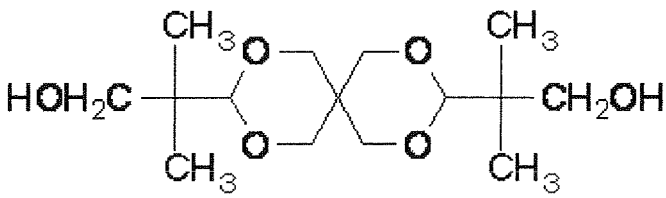

(3)

Examples of the dihydroxy compound represented by general formula (2) include isosorbide, isomannide, and isoidide, which are stereoisomers. These compounds may be used alone or in combination of two or more thereof.

Of those dihydroxy compounds, isosorbide is most preferred from the standpoints of easy availability, ease of production, optical properties, moldability, and carbon neutrality. Isosorbide is obtained by the dehydrating condensation of sorbitol, which is produced from various starches that are abundant resources derived from plants and are easily available.

The solid dihydroxy compound according to the invention preferably is solid at 25°C. The melting point thereof is preferably 60°C or higher and is preferably 220°C or lower. Incidentally, isosorbide, which is suitable for use as the solid dihydroxy compound according to the invention, has a melting point of about 66°C. Dihydroxy compounds having a melting point lower than 60°C are undesirable for the following reasons. Especially in transportation by ship beyond a tropic, there are cases where the ship heats up to a temperature close to 60°C. Furthermore, such dihydroxy compounds

during raw-material preparation are apt to unexpectedly suffer fusion bonding or cause clogging. On the other hand, use of a dihydroxy compound having a melting point exceeding 220°C results in troubles, such as an impaired color tone of the resultant polycarbonate, because a high temperature is necessary for raw-material preparation. The melting point of a dihydroxy compound can be determined from a melting peak top obtained when the dihydroxy compound is heated at 20°C/min with a differential scanning calorimeter (DSC).

The solid dihydroxy compound according to the invention may contain stabilizers, such as a reducing agent, antioxidant, free-oxygen absorber, light stabilizer, antacid, pH stabilizer, and heat stabilizer, so long as such stabilizers do not lessen the effects of the invention. It is preferred that the dihydroxy compound should contain basic stabilizers because the compound is apt to alter especially under acidic conditions.

Examples of the basic stabilizers include the hydroxides, carbonates, phosphates, phosphites, hypophosphites, borates, and fatty acid salts of metals in Group 1 or Group 2 of the long-form periodic table (Nomenclature of Inorganic Chemistry IUPAC Recommendations 2005) (hereinafter referred to simply as "Group 1" and "Group 2"). Examples thereof further include basic ammonium compounds such as tetramethylammonium hydroxide, tetraethylammonium hydroxide, tetrapropylammonium hydroxide, tetrabutylammonium hydroxide, trimethylethylammonium hydroxide, trimethylbenzylammonium hydroxide, trimethylphenylammonium hydroxide, triethylmethylammonium hydroxide, triethylbenzylammonium hydroxide, triethylphenylammonium hydroxide, tributylbenzylammonium hydroxide, tributylphenylammonium hydroxide, tetraphenylammonium hydroxide, benzyltriphenylammonium hydroxide, methyltriphenylammonium hydroxide, and butyltriphenylammonium hydroxide and amine compounds such as 4-aminopyridine, 2-aminopyridine, N,N-dimethyl-4-aminopyridine, 4-diethylaminopyridine, 2-hydroxypyridine, 2-methoxypyridine, 4-methoxypyridine, 2-dimethylaminoimidazole, 2-methoxyimidazole, imidazole, 2-mercaptoimidazole, 2-methylimidazole, and aminoquinoline. Of these, the phosphate or phosphite of sodium or potassium is preferred from the standpoints of the effect thereof and ease of the removal thereof by distillation which will be described later. Preferred of these are disodium hydrogen phosphate and disodium hydrogen phosphite.

The content of those basic stabilizers is not particularly limited. However, too low contents thereof produce no effect, while too high contents thereof may alter rather than stabilize the dihydroxy compound. Consequently, the content of the basic stabilizers is generally 0.0001-1% by weight, preferably 0.001-0.1% by weight, based on the solid dihydroxy compound according to the invention.

In case where the dihydroxy compound containing those basic stabilizers is used as a polycarbonate raw material without removing the stabilizers therefrom, the stabilizers themselves function as a polymerization catalyst, making it difficult to control polymerization rate and quality. It is therefore preferred to remove the basic stabilizers with an ion-exchange resin or by distillation, etc. before the dihydroxy compound is used.

The solid dihydroxy compound according to the invention which has been stored by the method of storage of the invention may have a formic acid content of 20 ppm by weight or lower, preferably 10 ppm by weight or lower, especially preferably 5 ppm by weight or lower. When the solid dihydroxy compound according to the invention which has such a formic acid content is used even without being subjected to a further purification treatment, a polycarbonate excellent in color tone and thermal stability can be produced without impairing polymerizability. Incidentally, the content of formic acid can be determined by ion chromatography.

After having been produced, a solid dihydroxy compound according to the invention is packed, in the form of a powder or molded pellets for facilitating handling, into a cargo container or a resin bag and/or paper bag and shipped in order to finally subject the dihydroxy compound to molding and/or processing, etc. The dihydroxy compound shipped, which is in the state of being packed in the container, is stored in a stock room or transported with a truck, ship, or the like. However, the solid dihydroxy compound according to the invention is apt to become a fusion-bonded mass, because this compound has a low melting point and is susceptible to deliquescence and the internal temperature of the container frequently rises during storage or transportation and because the compound is compacted by the load of the raw material itself.

The container to be used in the method of storage of the invention (hereinafter often referred to as "storage container") is not particularly limited so long as the container has sufficient pressure resistance and has gastightness. A suitable container can be selected according to the amount of the solid dihydroxy compound according to the invention to be stored and the purpose of storage. However, a metallic container, resinous container, fiber drum, flexible container, and paper bag are preferred. Preferred of these are a metallic container, resinous container, and fiber drum in the case of storing a large amount of the dihydroxy compound in a stock room or the like or transporting the dihydroxy compound with a ship or the like. This is because an excessive pressure is not directly applied to the solid dihydroxy compound according to the invention packed in lower ones of such storage containers stacked in multilayer arrangement.

In the case where flexible containers or paper bags are used as storage containers, it is necessary to regulate the pressure to be applied to the bottom of each storage container to 0.5 $kgf/cm^2$ or lower because the load of upper packages directly influences as stated above.

In the method of storage of the invention, it is essential that the pressure as measured at the bottom of the container should be 0.005-0.5 $kgf/cm^2$. The pressure is preferably 0.01-0.2 $kgf/cm^2$, and a suitable value thereof is

0.02-0.1 kgf/cm$^2$. Values of the pressure lower than 0.005 kgf/cm$^2$ are undesirable because the amount of the dihydroxy compound which can be packed into the container is too small. Values thereof exceeding 0.5 kgf/cm$^2$ are undesirable because it is impossible to inhibit the solid dihydroxy compound according to the invention from being compacted by the load of itself.

It is preferred that those storage containers should have an inner bag constituted of a resinous film from the standpoints of maintaining gastightness and preventing external contamination. It is more preferred that the containers should have an inner bag constituted of a resinous film having oxygen gas barrier properties from the standpoint of enhancing gastightness. As the resinous film having gas barrier properties, use can be made of films made of known resins having gas barrier properties, such as ethylene/vinyl alcohol copolymers, and resin films having an inorganic layer with gas barrier properties formed on a surface thereof.

Examples of the inorganic layer having gas barrier properties include films of metals, e.g., aluminum, formed by vapor deposition or the like and films of metal oxides, e.g., silica and alumina, formed by the sol-gel method, vapor deposition, or the like.

Resin films on which an inorganic substance having gas barrier properties has been vapor-deposited not only have excellent durability but also have an advantage that there is a wider choice of materials because a resin having no gas barrier properties is also usable as the resinous film serving as a base.

The thickness of the resinous film is not limited so long as sufficient gas barrier properties can be ensured. The thickness thereof is generally from 5 μm to 10 mm, preferably from 10 μm to 1,000 μm, especially preferably from 30 μm to 500 μm.

Storage conditions in the method of storage of the invention are explained below.

It is essential to the method of storage of the invention that the water content of the solid dihydroxy compound according to the invention, the internal temperature of the container, and the maximum pressure applied to the solid dihydroxy compound according to the invention should be regulated so as to be within given ranges.

In the method of storage of the invention, the water content of the solid dihydroxy compound according to the invention placed in a storage atmosphere is kept at 1.0% by weight or lower, preferably 0.5% by weight or lower, more preferably 0.2% by weight or lower. In case where the water content thereof is high, this solid dihydroxy compound tends to considerably deteriorate through dissolution and is apt to be compacted. Incidentally, the water content of the solid dihydroxy compound according to the invention can be determined by Karl Fischer's method.

The temperature at which the solid dihydroxy compound according to the invention is stored is 60°C or lower, preferably 40°C or lower, especially preferably 20°C or lower. By the method of storage of the invention, the solid dihydroxy compound according to the invention can be stored at temperatures up to 60°C, which is a relatively high temperature, without causing quality deterioration of the compound. At temperatures exceeding 60°C, however, there is a problem that the solid dihydroxy compound according to the invention is apt to suffer fusion bonding even when the compound is regulated so as to have the water content and load is regulated in the manner which will be described later.

There is no particular lower limit on the storage temperature. However, too low temperatures are disadvantageous from the standpoint of profitability because of the necessity of complicated management steps. Consequently, the storage temperature is generally -10°C or higher, especially preferably 0°C or higher.

Besides the storage conditions described above, other conditions may influence the quality of the solid dihydroxy compound according to the invention. In particular, oxygen concentration in the container frequently is a cause of the yellowing and reduced polymerizability of the solid dihydroxy compound according to the invention. Consequently, the oxygen concentration in the container is preferably 0.0001-10 vol%, especially preferably 0.001-1 vol%. Oxygen concentrations in the container lower than 0.0001 vol% are undesirable because it is practically difficult to attain such a concentration. Oxygen concentrations exceeding 10 vol% are undesirable because the solid dihydroxy compound according to the invention undergo a considerable decrease in quality.

Methods for regulating the oxygen concentration in the container are not particularly limited. However, the following methods are preferred because the methods are simple and effective: a method in which the solid-state dihydroxy compound is packed into a container having gas barrier properties and the atmosphere in this container is replaced with an inert gas, e.g., nitrogen; a method in which the compound is packed under vacuum; and a method in which the solid-state dihydroxy compound and a free-oxygen absorber are caused to coexist in the container while keeping the dihydroxy compound and the absorber not in contact with each other. Specifically, examples include a method in which the solid-state dihydroxy compound and a free-oxygen absorber packaged separated from the solid-state dihydroxy compound are caused to coexist in the container.

As the free-oxygen absorber, use can be made, for example, of free-oxygen absorbers including metallic ingredients represented by, e.g., an iron powder, ascorbic acid and salts thereof (including erythorbic acid, which is an isomer for ascorbic acid, and salts thereof), polyhydric alcohols such as glycerol, ethylene glycol, and propylene glycol, polyhydric phenols such as gallic acid, catechol, and pyrogallol, and substances having an unsaturated double bond or another readily oxidizable portion, such as unsaturated carbon and hydrogenated rubbers. However, free-oxygen absorbers including an iron powder or ascorbic acid as a component are preferred from the standpoint of ease of handling.

Materials for packaging such a free-oxygen absorber are not particularly limited so long as the materials are sufficiently permeable to oxygen. Use can be made of an oxygen-permeable resin film or a resin film having voids formed therein in net pattern arrangement.

According to the method of storage of the invention, the solid dihydroxy compound according to the invention can be stored over at least 30 days, preferably over 60 days or more, without causing compaction.

It is preferred that the solid dihydroxy compound according to the invention in a solid state should have an average bulk density of generally 200-1,000 kg/$m^3$, especially 300-900 kg/$m^3$, in particular 500-900 kg/$m^3$. The term "average bulk density of the solid dihydroxy compound according to the invention" means an average of the bulk densities of three 1-kg portions obtained by arbitrarily sampling the solid dihydroxy compound according to the invention. In case where the average bulk density thereof is lower than 200 kg/$m^3$, a stable feed rate is not obtained and this results in unevenness in polymerization reaction rate or forms a cause of dust explosion. When the average bulk density thereof exceeds 1,000 kg/$m^3$, there are cases where this solid dihydroxy compound becomes a fusion-bonded mass having a size and hardness which render the mass unsuitable for use as a raw material for polycarbonates. An increased cost is necessary for disaggregating the fusion-bonded mass. Such too high average bulk densities are hence undesirable. It is preferred that the solid dihydroxy compound according to the invention which has become a solid mass or fusion-bonded mass should be disaggregated with a disaggregating machine such as, e.g., a hammer mill, hammer breaker, nibbler, Fitz mill, jet mill, or rubber chopper to regulate the average bulk density thereof to a value within that range.

In the case of conducting disaggregation, it is preferred to prepare the dihydroxy compound so that the resultant disaggregation product contains, per kg, 20 or less solid masses having a maximum length of from 3 cm to 30 cm. The dihydroxy compound thus prepared has improved handleability in polycarbonate production and renders precise raw-material feeding possible.

(B) Method of Preparing Polycarbonate Raw Material

The method of preparing a polycarbonate raw material of the invention (hereinafter sometimes referred to as "method of preparation of the invention") is explained.

In the method of preparation of the invention, a dihydroxy compound according to the invention which has the structure represented by the following formula (1) in the molecule and is solid at 25°C is mixed with a molten carbonic acid diester under the conditions of a pressure of from 0.06 MPa to 0.20 MPa and a period of from 0.5 hours to 30 hours, and the resultant mixture is used as a raw material for a polycarbonate,
[Chem. 11]

$$-CH_2-O- \qquad (1)$$

(provided that the structure of formula (1) in which the oxygen atom is bonded to a hydrogen atom is excluded).

One of the features of the method of preparation of the invention resides in that a dihydroxy compound which has the structure represented by formula (1) in the molecule and is solid at 25°C is used as an essential component of a polycarbonate raw material.

The term "dihydroxy compound which is solid at 25°C" means a dihydroxy compound having a melting point higher than at least 25°C. This dihydroxy compound need not be liquid when used. This dihydroxy compound may be used in the form of a melt, solution, or suspension, for example, by using the compound at a temperature not lower than the melting point thereof or by using an appropriate solvent.

It is therefore preferred to use a compound which is solid at 25°C selected from the dihydroxy compounds having the structure represented by formula (1) which have been explained above with regard to the method of storage of the invention described above (solid dihydroxy compounds according to the invention).

Namely, examples of the dihydroxy compound according to the invention are the solid dihydroxy compounds according to the invention described above which are solid at 25°C. Specific examples thereof include compounds having a side chain including an aromatic ring and further having an aromatic-group-bonded ether group in the main chain, such as 9,9-bis(4-(2-hydroxyethoxy)phenyl)fluorene, 9,9-bis(4-(2-hydroxyethoxy)-3-methylphenyl)fluorene, 9,9-bis(4-(2-hydroxyethoxy)-3-isopropyl-phenyl)fluorene, 9,9-bis(4-(2-hydroxyethoxy)-3-isobutylphenyl)fluorene, 9,9-bis(4-(2-hydroxyethoxy)-3-tert-butylphenyl)fluorene, 9,9-bis(4-(2-hydroxyethoxy)-3-cyclo-hexylphenyl)fluorene, 9,9-bis(4-(2-hydroxyethoxy)-3-phenylphenyl)fluorene, 9,9-bis(4-(2-hydroxyethoxy)-3,5-dimethylphenyl)fluorene, 9,9-bis(4-(2-hydroxyethoxy)-3-tert-butyl-6-methylphenyl)fluorene, and 9,9-bis(4-(3-hydroxy-2,2-dimethylpropoxy)-phenyl)fluorene, bis(hydroxylalkoxyaryl)alkanes such as bis[4-(2-hydroxy-ethoxy)phenyl]methane, bis[4-(2-hydroxyethoxy)phenyl]diphenylmethane, 1,1-bis[4-(2-hydroxyethoxy)phenyl] ethane, 1,1-bis[4-(2-hydroxyethoxy)phenyl]-1-phenylethane, 2,2-bis[4-(2-hydroxyethoxy)phenyl]propane, 2,2-bis[4-(2-hydroxy-ethoxy)-3-methyl-phenyl]propane, 2,2-bis[3,5-dimethyl-4-(2-hydroxyethoxy)phenyl]propane, 1,1-bis[4-(2-hydroxyethoxy)-phenyl]-3,3,5-trimethylcyclohexane, 1,1-bis[4-(2-hydroxyethoxy)-phenyl]cyclohexane, 1,4-bis[4-(2-hydroxyethoxy)phenyl]cyclohexane, 1,3-bis[4-(2-hydroxyethoxy)phenyl]cyclohexane, 2,2-bis[4-(2-hydroxyethoxy)-3-phenylphenyl]-propane, 2,2-bis[(2-hydroxyethoxy)-3-isopropylphenyl]propane, 2,2-bis[3-tert-butyl-4-(2-hydroxyethoxy)phenyl]propane, 2,2-bis[4-(2-hydroxyethoxy)phenyl]butane, 2,2-bis[4-(2-hydroxyethoxy)phenyl]-4-methylpentane, 2,2-bis[4-(2-hydroxyethoxy)phenyl]-octane, 1,1-bis[4-(2-hydroxyethoxy)phenyl]decane, 2,2-bis[3-bromo-4-(2-hydroxy-ethoxy)phenyl]propane, and 2,2-bis[3-cyclohexyl-4-(2-hydroxyethoxy)phenyl]propane, bis(hydroxyalkoxyaryl)cycloalkanes such as 1,1-bis[4-(2-hydroxyethoxy)phenyl]-cyclohexane, 1,1-bis[3-cyclohexyl-4-(2-hydroxyethoxy)phenyl]cyclohexane, and 1,1-bis[4-(2-hydroxyethoxy)phenyl]cyclopentane, dihydroxyalkoxydiaryl ethers such as 4,4'-bis(2-hydroxyethyl)diphenyl ether and 4,4'-bis(2-hydroxyethoxy)-3,3'-dimethyldiphenyl ether, bishydroxyalkoxyaryl sulfides such as 4,4'-bis(2-hydroxyethoxyphenyl) sulfide and 4,4'-bis[4-(2-dihydroxyethoxy)-3-methylphenyl] sulfide, bishydroxyalkoxyaryl sulfoxides such as 4,4'-bis(2-hydroxyethoxyphenyl) sulfoxide and 4,4'-bis[4-(2-dihydroxyethoxy)-3-methylphenyl] sulfoxide, bishydroxyalkoxyaryl sulfones such as 4,4'-bis(2-hydroxyethoxyphenyl) sulfone and 4,4'-bis[4-(2-dihydroxyethoxy)-3-methylphenyl]sulfone, 1,4-bishydroxyethoxybenzene, 1,3-bishydroxyethoxybenzene, 1,2-bishydroxyethoxybenzene, 1,3-bis[2-[4-(2-hydroxy-ethoxy)phenyl]propyl]benzene, 1,4-bis[2-[4-(2-hydroxyethoxy)phenyl]propyl]benzene, 4,4'-bis(2-hydroxyethoxy)biphenyl, 1,3-bis[4-(2-hydroxyethoxy)phenyl]-5,7-dimethyl-adamantane, anhydrous sugar alcohols represented by a dihydroxy compound represented by the following formula (2), and compounds having a cyclic ether structure, such as the spiro-glycol represented by the following general formula (3). These compounds may be used alone or in combination of two or more thereof.

[Chem. 12]

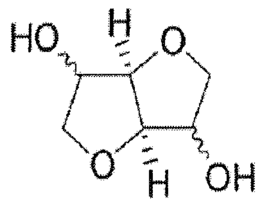

(2)

[Chem. 13]

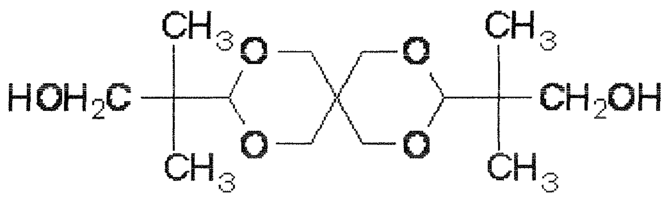

(3)

Examples of the dihydroxy compound represented by general formula (2) include isosorbide, isomannide, and isoidide, which are stereoisomers. These compounds may be used alone or in combination of two or more thereof.

Of those dihydroxy compounds, isosorbide is most preferred from the standpoints of easy availability, ease of production, optical properties, moldability, and carbon neutrality. Isosorbide is obtained by the dehydrating condensation of sorbitol, which is produced from various starches that are abundant resources derived from plants and are easily available.

The polycarbonate raw material in the invention may include structural units derived from dihydroxy compounds other than the dihydroxy compound according to the invention (hereinafter sometimes referred to as "other dihydroxy compounds"). Examples of the other dihydroxy compounds include aliphatic diol compounds such as ethylene glycol, 1,3-propanediol, 1,2-propanediol, 1,4-butanediol, 1,3-butanediol, 1,2-butanediol, 1,5-heptanediol, and 1,6-hexanediol, alicyclic dihydroxy compounds such as 1,2-cyclohexanedimethanol, 1,3-cyclohexanedimethanol, 1,4-cyclohexanedimethanol, tricyclodecanedimethanol, pentacyclopentadecanedimethanol, 2,6-decalindimethanol, 1,5-decalindimethanol, 2,3-decalindimethanol, 2,3-norbornanedimethanol, 2,5-norbornanedimethanol, and 1,3-adamantanedimethanol, and aromatic bisphenols such as 2,2-bis(4-hydroxyphenyl)propane [= bisphenol A], 2,2-bis(4-hydroxy-3,5-dimethylphenyl)propane, 2,2-bis(4-hydroxy-3,5-diethylphenyl)propane, 2,2-bis(4-hydroxy(3,5-diphenyl)phenyl)propane, 2,2-bis(4-hydroxy-3,5-dibromophenyl)propane, 2,2-bis(4-hydroxyphenyl)pentane, 2,4' -dihydroxydiphenylmethane, bis(4-hydroxyphenyl)methane, bis(4-hydroxy-5-nitrophenyl)methane, 1,1-bis(4-hydroxyphenyl)-ethane, 3,3-bis(4-hydroxyphenyl)pentane, 1,1-bis(4-hydroxyphenyl)cyclohexane, bis(4-hydroxyphenyl) sulfone, 2,4'-dihydroxydiphenyl sulfone, bis(4-hydroxyphenyl) sulfide, 4,4'-dihydroxydiphenyl ether, 4,4'-dihydroxy-3,3'-dichlorodiphenyl ether, 9,9-bis(4-(2-hydroxyethoxy-2-methyl)phenyl)fluorene, 9,9-bis(4-hydroxyphenyl)fluorene, and 9,9-bis(4-hydroxy-2-methylphenyl)fluorene.

Preferred of these from the standpoints of optical properties, availability, and handleability are 1,4-cyclohexanedimethanol, 1,3-cyclohexanedimethanol, 1,2-cyclohexanedimethanol, and tricyclodecanedimethanol.

By using such other dihydroxy compounds, the effects of improving flexibility, enhancing heat resistance, improving moldability, etc. may be obtained. However, when the proportion of structural units derived from such other dihydroxy compounds is too high, there are cases where the inherent optical properties decrease or heat resistance decreases. In addition, the effects of the invention are lessened. Consequently, it is preferred that the proportion of the dihydroxy compound according to the invention to the dihydroxy compound ingredient (sum of the dihydroxy compound according to the invention and the other dihydroxy compounds) should be 20% by mole or higher, preferably 30% by mole or higher, especially preferably 50% by mole or higher.

It is essential to the method of preparation of the invention that the dihydroxy compound according to the invention should be solid at 25°C. It is preferred that the melting point thereof should be preferably 40°C or higher, more preferably 50°C or higher, especially preferably 60°C or higher, and be, in particular, 220°C or lower, preferably 150°C or lower, more preferably 120°C or lower, especially 100°C or lower. In case where the melting point thereof is lower than 40°C, this dihydroxy compound during raw-material preparation is apt to unexpectedly suffer fusion bonding or cause clogging. On the other hand, use of a dihydroxy compound having a melting point exceeding 220°C results in troubles, such as an impaired color tone of the resultant polycarbonate, because a high temperature is necessary for raw-material preparation. The melting point of a dihydroxy compound can be determined from the top of a melting peak appearing when the dihydroxy compound is heated at 20°C/min with a differential scanning calorimeter (DSC).

The dihydroxy compound according to the invention may contain stabilizers, such as a reducing agent, antioxidant, free-oxygen absorber, light stabilizer, antacid, pH stabilizer, and heat stabilizer. It is preferred that the dihydroxy compound should contain basic stabilizers because the compound is apt to alter especially under acidic conditions. Examples of the basic stabilizers include the hydroxides, carbonates, phosphates, phosphites, hypophosphites, borates, and fatty acid salts of metals in Group 1 or Group 2 of the long-form periodic table (Nomenclature of Inorganic Chemistry IUPAC Recommendations 2005) (hereinafter referred to simply as "Group 1" and "Group 2"). Examples thereof further include basic ammonium compounds such as tetramethylammonium hydroxide, tetraethylammonium hydroxide, tetrapropylammonium hydroxide, tetrabutylammonium hydroxide, trimethylethylammonium hydroxide, trimethylbenzylammonium hydroxide, trimethylphenylammonium hydroxide, triethylmethylammonium hydroxide, triethylbenzylammonium hydroxide, triethylphenylammonium hydroxide, tributylbenzylammonium hydroxide, tributylphenylammonium hydroxide, tetraphenylammonium hydroxide, benzyltriphenylammonium hydroxide, methyltriphenylammonium hydroxide, and butyltriphenylammonium hydroxide and amine compounds such as 4-aminopyridine, 2-aminopyridine, N,N-dimethyl-4-aminopyridine, 4-diethylaminopyridine, 2-hydroxypyridine, 2-methoxypyridine, 4-methoxypyridine, 2-dimethylaminoimidazole, 2-methoxyimidazole, imidazole, 2-mercaptoimidazole, 2-methylimidazole, and aminoquinoline. Of these, the phosphate or phosphite of sodium or potassium is preferred from the standpoints of the effect thereof and ease of the removal thereof by distillation which will be described later. Preferred of these are disodium hydrogen phosphate and disodium hydrogen phosphite.

The content of those basic stabilizers is not particularly limited. However, too low contents thereof produce no effect, while too high contents thereof may alter rather than stabilize the dihydroxy compound. Consequently, the content of the basic stabilizers is generally 0.0001-1% by weight, preferably 0.001-0.1% by weight, based on the dihydroxy compound according to the invention.

In case where the dihydroxy compound containing those basic stabilizers is used as a polycarbonate raw material without removing the stabilizers therefrom, the stabilizers themselves function as a polymerization catalyst, making it difficult to control polymerization rate and quality. It is therefore preferred to remove the basic stabilizers with an ion-exchange resin or by distillation, etc. before the dihydroxy compound is used.

In the case where the dihydroxy compound according to the invention is one having a cyclic ether structure, e.g., isosorbide, this dihydroxy compound is apt to be gradually oxidized by oxygen. It is therefore important in handling for storage or during production that decomposition by oxygen should be prevented, for example, by preventing water inclusion or by using a free-oxygen absorber or handling the dihydroxy compound in a nitrogen atmosphere. Oxidation of isosorbide generates decomposition products including formic acid. In case where isosorbide containing these decomposition products, for example, is used to produce a polycarbonate, not only the resultant polycarbonate has a color and considerably impaired properties, but also the decomposition products influence the polymerization reaction, making it impossible to obtain a high-molecular polymer. Use of such isosorbide is hence undesirable.

It is preferred that the dihydroxy compound according to the invention should be purified by distillation, before being used as a polycarbonate raw material, in order to obtain the dihydroxy compound which does not contain those oxidation decomposition products and to remove the basic stabilizers. The distillation in this case may be simple distillation or continuous distillation, and is not particularly limited. In particular, it is preferred to form an inert gas atmosphere, such as argon or nitrogen, and then conduct distillation at a reduced pressure. From the standpoint of inhibiting thermal alteration as far as possible, it is preferred to conduct the distillation at 250°C or lower, preferably 200°C or lower, especially 180°C or lower.

By purification by such distillation, the formic acid content of the dihydroxy compound according to the invention may be reduced to 20 ppm by weight or lower, preferably 10 ppm by weight or lower, especially preferably 5 ppm by weight or lower.

As a result, a polycarbonate excellent in color tone and thermal stability can be produced without impairing polymerizability. The content of formic acid is determined by ion chromatography.

In the method of preparation of the invention, the dihydroxy compounds described above are mixed with a carbonic acid diester under the conditions of a pressure of from 0.06 MPa to 0.20 MPa and a period of from 0.5 hours to 30 hours, and the resultant mixture is used as a raw material for a polycarbonate. Using the raw material prepared, a polycarbonate can be produced by the so-called transesterification method. Examples of the carbonic acid diester to be used in the method of preparation of the invention generally include diesters represented by the following general formula (4).

[Chem. 14]

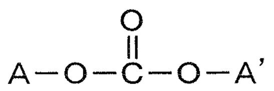

(4)

(In general formula (4), A and A' are an aliphatic group which has 1-18 carbon atoms and may have a substituent or an aromatic group which may have a substituent, provided that A and A' may be the same or different.)

Examples of the carbonic acid diesters represented by general formula (4) include diphenyl carbonate, substituted diphenyl carbonates represented by ditolyl carbonate, dimethyl carbonate, diethyl carbonate, and di-t-butyl carbonate. However, diphenyl carbonate and substituted diphenyl carbonates are preferred. Especially preferred is diphenyl carbonate. One of these carbonic acid diesters may be used alone, or a mixture of two or more thereof may be used. There are cases where carbonic acid esters contain impurities such as, e.g., chloride ions. It is therefore preferred to use a carbonic acid diester purified by, e.g., distillation.

It is essential to the method of preparation of the invention that the dihydroxy compound according to the invention should be mixed with a molten carbonic acid diester and the resultant mixture be used as a raw material for a polycarbonate.

The dihydroxy compound according to the invention may be mixed, in the original solid state, with a molten carbonic acid diester, or may be brought into the state of a melt, solution, or suspension and then mixed with a molten carbonic acid diester. However, from the standpoints of suitability for operation and feeding stability, it is preferred that the dihydroxy compound should be brought into the state of a melt, solution, or suspension and then mixed with a molten carbonic acid diester. From the standpoint of quantitativeness, it is preferred to mix the dihydroxy compound as a melt.

From the standpoint of preventing deterioration, it is preferred that the operation for melting, dissolving, or suspending the dihydroxy compound according to the invention should be conducted in an atmosphere having an oxygen concentration of 10 vol% or lower, preferably 0.0001-10 vol%, in particular 0.0001-5 vol%, especially 0.0001-1 vol%.

In the case of mixing the dihydroxy compound according to the invention in a solid state with a molten carbonic acid diester, use may be made of a method in which the solid-state dihydroxy compound is fed to the molten carbonic acid diester, a method in which the molten carbonic acid diester is fed to the solid-state dihydroxy compound, or a method in which the two ingredients are simultaneously fed. However, from the standpoint of the effects of the invention, it is preferred to feed the solid-state dihydroxy compound to the molten carbonic acid diester.

Diphenyl carbonate, which is a typical carbonic acid diester, has a melting point of about 80°C. When a feed material having poor thermal stability, such as the dihydroxy compound according to the invention, is used, a raw material can be prepared at a relatively low temperature by dissolving this compound in a molten carbonic acid diester. As a result, polymerizability can be inhibited from being impaired by thermal deterioration and the polycarbonate as a final product can be inhibited from having a color. Furthermore, when a carbonic acid diester is mixed beforehand with a dihydroxy compound having a low melting point, this mixture can have a lower melting point. To mix this mixture with the dihydroxy compound according to the invention is effective from the standpoint of reducing thermal history.

The temperature at which the dihydroxy compound according to the invention is mixed with a molten carbonic acid diester is generally 70°C or higher, preferably 80°C or higher, more preferably 90°C or higher. The upper limit thereof is generally 240°C or lower, preferably 200°C or lower, more preferably 150°C or lower. In particular, a temperature of from 100°C to 120°C is suitable. In case where the temperature is too low, the rate of dissolution is low and this not only necessitates excessive equipment but also often results in troubles such as solidification. Too high temperatures result in thermal deterioration of the dihydroxy compound.

From the standpoint of preventing deterioration, it is preferred that the operation for mixing the dihydroxy compound according to the invention with a molten carbonic acid diester should be conducted in an atmosphere having

an oxygen concentration of 10 vol% or lower, preferably 0.0001-10 vol%, in particular 0.0001-5 vol%, especially 0.0001-1 vol%.

The rate at which the dihydroxy compound according to the invention is mixed with a molten carbonic acid diester is not particularly limited. However, when the weight of the carbonic acid diester melt, in terms of parts by weight, is expressed by A and the rate of feeding the dihydroxy compound according to the invention, in terms of parts by weight per hour, is expressed by B, then it is preferred that A and B should satisfy B≤6A, more preferably B≤4A, especially B≤3A. In this connection, in the case where the carbonic acid diester has been mixed with other monomers, etc., A represents the amount in parts by weight of the carbonic acid diester only. In the case where the dihydroxy compound according to the invention has been brought into the state of a solution or suspension, the net rate of feeding the dihydroxy compound excluding the solvent or dispersion medium, in terms of parts by weight per hour, is expressed by B.

In the case of continuous feeding, that feed rate is the average of the feed rates therein. In the case of intermittent feeding, a feed rate is determined by dividing the weight of the dihydroxy compound which was fed in the preceding feeding operation by the time period from initiation of the preceding feeding operation to initiation of the succeeding feeding operation.

In the case where the dihydroxy compound according to the invention is directly fed to a molten carbonic acid diester, the dihydroxy compound according to the invention does not readily dissolve when the feed rate is too high. This operation hence gives an uneven raw material and is a cause of impartation of an unnecessary heat history. Also in the case where the dihydroxy compound according to the invention is fed as a melt, solution, or suspension at too high a feed rate, heat absorption occurs upon mixing with the carbonic acid diester. As a result, the contents may solidify to cause an apparatus trouble. On the other hand, too low feed rates necessitate an excessively large apparatus or result in a large heat history.

It is preferred that the dihydroxy compound according to the invention in a solid state should have an average bulk density of generally 200-1,000 kg/$m^3$, especially 300-900 kg/$m^3$, in particular 500-900 kg/$m^3$. The term average bulk density means an average of the bulk densities of three 1-kg portions obtained by arbitrarily sampling the solid-state dihydroxy compound. In case where the average bulk density thereof is lower than 200 kg/$m^3$, a stable feed rate is not obtained and this results in unevenness in polymerization reaction rate or forms a cause of dust explosion. There are cases where the dihydroxy compound according to the invention partly becomes massive due to the influence of a slight amount of water, stabilizers, etc. contained therein or because of the method of transportation, etc., and thereby comes to have an average bulk density exceeding 1,000 kg/$m^3$. In such cases, it is preferred to crush the solid mass with a hammer mill, hammer breaker, nibbler, Fitz mill, jet mill, rubber chopper, or the like to regulate the average bulk density of the dihydroxy compound to a value within that range.

It is also preferred that the solid-state dihydroxy compound according to the invention should be received with a hopper having a bottom cone angle of 120 degrees or smaller, preferably 90 degrees or smaller, more preferably 70 degrees or smaller, and that the atmosphere should be regulated, while maintaining the solid state, so as to have an oxygen concentration of 10 vol% or lower, preferably 5 vol% or lower, especially preferably 2 vol% or lower, by, e.g., replacement with an inert gas, before the dihydroxy compound is brought into the state of a melt, solution, or suspension. In the case where the solid-state dihydroxy compound according to the invention is melted, it is preferred that a small amount of a melt of the dihydroxy compound should be prepared beforehand and the solid-state dihydroxy compound be gradually added thereto in order to minimize thermal deterioration.

With respect to the temperature at which the solid-state dihydroxy compound according to the invention is melted, it is preferred to regulate the internal temperature so as to be in the range of from the melting point of the dihydroxy compound to (melting point + 50°C), especially from the melting point to (melting point + 30°C), in particular from the melting point to (melting point + 20°C), in order to minimize thermal deterioration and prevent the contents from solidifying.

The rate at which the solid-state dihydroxy compound according to the invention is fed is not particularly limited. However, when the weight of the dihydroxy compound melted beforehand, in terms of parts by weight, is expressed by C and the rate of feeding the solid-state dihydroxy compound, in terms of parts by weight per hour, is expressed by D, then it is preferred that C and D should satisfy D≤6C, more preferably D≤4C, especially D≤3C. Too high feed rates may result in solidification of the contents because the solid-state dihydroxy compound according to the invention frequently absorbs heat upon melting. As a result, the solidification forms a cause of an apparatus trouble or of impartation of an unnecessary heat history. Too low feed rates necessitate an excessively large apparatus or result in a large heat history.

In the case of continuous feeding, that feed rate is the average of the feed rates therein. In the case of intermittent feeding, a feed rate is determined by dividing the weight of the solid-state dihydroxy compound which was fed in the preceding feeding operation by the time period from initiation of the preceding feeding operation to initiation of the succeeding feeding operation.

In the case where the dihydroxy compound according to the invention has a melting point of 150°C or lower, especially 100°C or lower, it is preferred to mix this dihydroxy compound in the form of a melt with a carbonic acid diester, because this dihydroxy compound can be handled at low temperatures which are less apt to cause thermal deterioration.

It is also preferred that the dihydroxy compound should be purified by distillation before being subjected to polymerization as stated hereinabove.

In the case where the dihydroxy compound according to the invention has a high melting point, it is preferred that this dihydroxy compound should be mixed with a carbonic acid diester after the dihydroxy compound is brought into a solution or suspension state. There are no particular limitations on solvents or dispersion media usable in this case. However, water is preferred from the standpoints of ease of separation/removal in the polymerization step and minimizing an influence on polycarbonate quality.

Also in the case where the dihydroxy compound according to the invention is mixed with a molten carbonic acid diester after having been brought into the state of a melt, solution, or suspension, the rate of feeding preferably is the same as described above. Namely, when the weight of the carbonic acid diester melt, in terms of parts by weight, is expressed by A and the rate of feeding the dihydroxy compound according to the invention, in terms of parts by weight per hour, is expressed by B, then it is preferred that A and B should satisfy $B \leq 6A$, more preferably $B \leq 4A$, especially $B \leq 3A$. In this connection, in the case where the carbonic acid diester has been mixed with other monomers, etc., A represents the amount in parts by weight of the carbonic acid diester only. In the case where the dihydroxy compound according to the invention has been brought into the state of a solution or suspension, the net rate of feeding the dihydroxy compound excluding the solvent or dispersion medium, in terms of parts by weight per hour, is expressed by B.

In general, an endothermic phenomenon occurs when the dihydroxy compound according to the invention is mixed with a carbonic acid diester. Because of this, two high feed rates may result in precipitation or solidification of the contents.

In the method of preparation of the invention, the other dihydroxy compounds described above can also be mixed with the carbonic acid diester in the same manner as for the dihydroxy compound according to the invention.

In the method of preparation of the invention, it is preferred that the carbonic acid diester should be used in a molar ratio of from 0.90 to 1.20, in terms of the molar ratio of the carbonic acid diester to the whole dihydroxy compound ingredient to be subjected to reaction. The molar ratio thereof is more preferably from 0.95 to 1.10. In case where the molar ratio thereof is lower than 0.90, the polycarbonate produced has an increased amount of terminal OH groups and hence has impaired thermal stability or a desired high-molecular polymer is not obtained. In case where the molar ratio thereof is higher than 1.20, the rate of transesterification reaction decreases when the reaction is conducted under the same conditions, and a polycarbonate having a desired molecular weight is difficult to produce. In addition, the polycarbonate produced has an increased content of the residual carbonic acid diester, and this residual carbonic acid diester is a cause of odor during molding or in molded articles. Such too high molar ratios are hence undesirable.

A preferred embodiment of the invention is explained below by reference to drawings. However, the invention should not be construed as being limited to the embodiment.

In Fig. 1, numeral 1 denotes a receiving hopper for a solid-state dihydroxy compound, 2 a weigh feeder, 3 a stirring tank equipped with a jacket, and 4 a discharge line.

The solid-state dihydroxy compound is crushed according to need and fed to the receiving hopper (1). The receiving hopper (1) has a bottom cone angle of preferably 120 degrees or smaller, and is equipped with an apparatus (not shown) which evacuates the inside of the hopper (1) and replaces the atmosphere with nitrogen. After reception of the solid-state dihydroxy compound, evacuation and replacement with nitrogen are repeated to reduce the oxygen concentration within the hopper. The atmosphere in the stirring tank (3) is replaced beforehand with nitrogen to keep the oxygen concentration therein at 1 vol% or lower. The stirring tank (3) in this state is heated, and a molten dihydroxy compound is placed therein as a first liquid feed. Subsequently, while regulating the stirring tank (3) so as to have a temperature within a given range, the solid-state dihydroxy compound is fed with the weigh feeder (2). In this operation, stirring or nitrogen bubbling is conducted according to need to evenly melt the contents. After completion of the melting, the melt is fed to a next step, preferably a distillation/purification step, through the discharge line (4). In the case where the dihydroxy compound melts at a sufficiently high rate, use may be made of a method in which the solid-state dihydroxy compound is continuously fed and the resultant melt is continuously discharged simultaneously with the feeding.

The dihydroxy compound which has been melted and optionally purified by distillation is sent to a storage tank (5) equipped with a heat-medium jacket through a line (5A) as shown in Fig. 2. Meanwhile, a carbonic acid diester externally fed in a molten state is sent through a line (6A) to a storage tank (6) equipped with a heat-medium jacket, and is sent in a given amount through a line (6B) to a raw-material preparation tank (9) equipped with a heat-medium jacket and a stirrer while measuring the amount of the carbonic acid diester with a constant delivery pump (not shown) or flow meter (not shown). The molten-state dihydroxy compound is sent in a given amount from the storage tank (5) to the raw-material preparation tank (9) while measuring the amount thereof with a constant delivery pump (not shown) or flow meter (not shown). This molten-state dihydroxy compound is evenly mixed with the molten carbonic acid diester by means of the stirrer while regulating the internal temperature with the jacket, and the resultant mixture is used as a raw material for a polycarbonate. A plurality of storage tanks (5) may be disposed to feed other dihydroxy compounds. According to need, the solid-state dihydroxy compound is received with a hopper (7) and, after optional replacement with an inert gas, this dihydroxy compound is fed to the carbonic acid diester within the raw-material preparation tank

through a line (7A) with a weigh feeder (8) and dissolved in and mixed with the carbonic acid diester. The raw material which has been prepared is analyzed, according to need, for molar ratio between the dihydroxy compound and the carbonic acid diester. Thereafter, the raw material is sent through a line (9A) to a raw-material storage tank (10) equipped with a jacket and then fed to a polymerization tank through a line (10A). The lines (5A), (5B), (6A), (6B), (9A), and (10A) are thermally insulated or heated in such a degree that the contents do not solidify, and a filter for removing foreign matter is disposed in the line (10A) according to need. The temperatures at which these lines are kept by thermal insulation or heating may be determined while taking account of the kinds and amounts of the monomers as the contents. For avoiding solidification of the contents, it is necessary that the temperature for each line should be not lower than the melting point of the monomer or monomer mixture as the contents. From the standpoint of avoiding alteration of the contents, the temperature at which each line is kept by thermal insulation or heating is preferably not higher than (the melting point + 100°C), more preferably not higher than (the melting point + 50°C), especially preferably not higher than (the melting point + 30°C). The mixing of the molten carbonic acid diester with the dihydroxy compound may be conducted batchwise or continuously.

A polymerization catalyst may be added to the raw-material preparation tank (9) or raw-material storage tank (10) or may be directly added to the polymerization tank. However, from the standpoints of stable feeding and polymerization control, it is preferred to dispose a catalyst feed line (not shown) somewhere in the line (10A) and to feed a catalyst through the catalyst feed line.

(C) Method of Producing Polycarbonate

The method of producing a polycarbonate of the invention is explained below.

In the method of producing a polycarbonate of the invention, a dihydroxy compound ingredient at least including a dihydroxy compound having the structure represented by the following formula (1) in the molecule and a carbonic acid diester are subjected as raw materials to transesterification to produce a polycarbonate.

It is preferred to use, as a raw material for a polycarbonate, either (A) the dihydroxy compound obtained by the method of storage of the invention described above or (B) the raw material including a dihydroxy compound and a carbonic acid diester and obtained by the method of preparing a polycarbonate raw material of the invention described above. However, usable raw materials are not limited to these.

The method of producing a polycarbonate of the invention which is characterized in that at least a dihydroxy compound having the structure represented by formula (1) is used as an essential ingredient and is mixed for a given time period at a specific temperature and a specific pressure is explained below as an especially preferred method among the method of polycarbonate production of the invention. However, the invention should not be construed as being limited thereto.

The method of producing a polycarbonate of the invention (hereinafter often referred to as "method of production of the invention") is a method of producing a polycarbonate from a dihydroxy compound ingredient at least including a dihydroxy compound having the structure represented by the following formula (1) in the molecule and from a carbonic acid diester, and is characterized by mixing the dihydroxy compound having the structure represented by the following formula (1) in the molecule (hereinafter often referred to as "dihydroxy compound (I)") with the carbonic acid diester in a molten state at a temperature of from 70°C to 240°C and a pressure of from 0.06 MPa to 0.20 MPa for a period of from 0.5 hours to 30 hours and then causing a transesterification reaction to proceed at a pressure lower than 0.06 MPa to produce a polycarbonate.

In the method of production of the invention, the term "dihydroxy compound ingredient" means the sum of all dihydroxy compounds serving as a raw material for a polycarbonate. The term "molten state" indicates the molten state of a raw-material monomer having a temperature not lower than the melting point thereof. However, the term does not especially indicate the necessity of a heat treatment. For example, carbonic acid diesters which are liquid at ordinary temperature are used as they are without undergoing a heat treatment. In the method of production of the invention, the term "pressure" indicates the so-called absolute pressure, which is based of vacuum.

[Chem. 15]

$$-CH_2-O- \qquad (1)$$

(The structure of formula (1) in which the oxygen atom is bonded to a hydrogen atom is excluded.)

In the method of production of the invention, the temperature at which the ingredients are mixed together (hereinafter sometimes referred to as "mixing temperature") is generally 70-240°C, preferably 80-150°C, especially preferably 90-120°C. When the mixing temperature is lower than 70°C, the ingredients mix together at a low rate and there is a possibility that precipitation of the raw-material monomer(s) (the dihydroxy compound ingredient and/or the carbonic acid diester) might occur. On the other hand, mixing temperatures exceeding 240°C are undesirable because such high temperatures cause the dihydroxy compound to deteriorate thermally. When the mixing temperature is, in

particular, 80-120°C, the dihydroxy compound ingredient is evenly mixed with the carbonic acid diester in a short time period without undergoing thermal deterioration.

The internal pressure of the tank during mixing (hereinafter sometimes referred to as "mixing pressure") is generally 0.06-0.20 MPa, preferably 0.09-0.12 MPa.

In case where the mixing pressure is lower than 0.06 MPa, monomer volatilization occurs and this results in a shifting in raw-material molar ratio, making it impossible to obtain a polymer having a sufficient molecular weight, or causes clogging of a vent part, etc. On the other hand, in case where the mixing pressure exceeds 0.20 MPa, not only the raw-material preparation tank but also the apparatus including the equipments is required to have a pressure-resistant structure, resulting in an excessively large apparatus size, and a complicated operation is necessary.

The mixing time for evenly mixing the raw-material monomers is from 0.5 hours to 30 hours, preferably 1.0-20 hours, especially preferably 1.0-10 hours. The term "mixing time" in the invention has the following meanings. When the monomers are mixed batchwise, that term means the time period from the feeding of the dihydroxy compound and the carbonic acid diester to a tank equipped with a stirrer to the discharge of the resultant mixture from the tank. When the ingredients are mixed continuously, that term means an average residence time from the feeding of the dihydroxy compound and the carbonic acid diester to a tank equipped with a stirrer to the discharge of the resultant mixture from the tank.

Whether or not the dihydroxy compound ingredient can be evenly mixed with a carbonic acid diester by the constitution of the method of production of the invention has not become completely clear. However, it is thought that when the dihydroxy compound ingredient is mixed with a carbonic acid diester for a given time period (from 0.5 hours to 30 hours) at the temperature (70-240°C) and pressure (0.06-0.20 MPa), then part of the dihydroxy compound (I) reacts with the carbonic acid diester to yield an oligomer and this oligomer functions as a compatibilizing agent to accelerate the mixing the dihydroxy compound ingredient with the carbonic acid diester. Consequently, in case where the mixing temperature, mixing pressure, or mixing time is outside the range according to the invention, the raw materials are not evenly mixed and it becomes difficult to stably conduct the polymerization reaction. Especially when the raw-material mixture is continuously fed from the raw-material mixing layer to a reaction tank, one ingredient is excessively fed and, hence, it becomes difficult to produce a polycarbonate having a desired molecular weight.

As the dihydroxy compound (I), use is made of a dihydroxy compound at least having the structure represented by general formula (1) in the molecule. Examples thereof are the same as the examples of the solid dihydroxy compound according to the invention shown above. One of these dihydroxy compounds may be used alone, or a combination of two or more thereof may be used.

Of such dihydroxy compounds (I), isosorbide is most preferred from the standpoints of easy availability, ease of production, optical properties, moldability, and carbon neutrality. Isosorbide is obtained by the dehydrating condensation of sorbitol, which is produced from various starches that are abundant resources derived from plants and are easily available. From the standpoint of optical properties, 9,9-bis(4-(2-hydroxyethoxy)phenyl)fluorene is preferred.

The dihydroxy compound (I) usually is solid at 25°C. It is preferred that the melting point thereof should be preferably 40°C or higher, more preferably 50°C or higher, especially preferably 60°C or higher, and be, in particular, 220°C or lower, preferably 150°C or lower, more preferably 120°C or lower, especially 100°C or lower. In case where the melting point thereof is lower than 40°C, this dihydroxy compound during raw-material preparation is apt to unexpectedly suffer fusion bonding or cause clogging. On the other hand, use of a dihydroxy compound having a melting point exceeding 220°C results in troubles, such as an impaired color tone of the resultant polycarbonate, because a high temperature is necessary for raw-material preparation. The melting point of a dihydroxy compound can be determined from the top of a melting peak appearing when the dihydroxy compound is heated at 20°C/min with a differential scanning calorimeter (DSC).

Like the solid dihydroxy compound according to the invention, the dihydroxy compound (I) may contain stabilizers, such as a reducing agent, antioxidant, free-oxygen absorber, light stabilizer, antacid, pH stabilizer, and heat stabilizer. It is preferred that the dihydroxy compound (I) should contain basic stabilizers because the compound is apt to alter especially under acidic conditions. As the basic stabilizers, use can be made of the same basic stabilizers as those used for the solid dihydroxy compound according to the invention. Of these, the phosphate or phosphite of sodium or potassium is preferred from the standpoints of the effect thereof and ease of the removal thereof by distillation which will be described later. Preferred of these are disodium hydrogen phosphate and disodium hydrogen phosphite.

The content of those basic stabilizers is not particularly limited. However, too low contents thereof produce no effect, while too high contents thereof may alter rather than stabilize the dihydroxy compound. Consequently, it is generally preferred to use the basic stabilizers in the same proportion by weight as for the solid dihydroxy compound according to the invention.

In case where the dihydroxy compound (I) containing those basic stabilizers is used as a polycarbonate raw material without removing the stabilizers therefrom, the stabilizers themselves function as a polymerization catalyst, making it difficult to control polymerization rate and quality. It is therefore preferred to remove the basic stabilizers with an ion-exchange resin or by distillation, etc. before the dihydroxy compound (I) is used.

In the case where the dihydroxy compound (I) is one having a cyclic ether structure, e.g., isosorbide, this dihydroxy compound is apt to be gradually oxidized by oxygen. It is therefore preferred that this dihydroxy compound (I) should be handled under conditions such as those described above with regard to the case where the solid dihydroxy compound according to the invention is a dihydroxy compound having a cyclic ether structure, e.g., isosorbide.

In the method of production of the invention, the dihydroxy compound ingredient for a polycarbonate raw material may include an aliphatic and/or an alicyclic dihydroxy compound (hereinafter often referred to as "dihydroxy compound (II)") besides the dihydroxy compound (I). Use of this dihydroxy compound (II) is also preferred.

The dihydroxy compound (II) is compatible with the dihydroxy compound (I) and carbonic acid diester which are other polycarbonate raw materials according to the invention. The dihydroxy compound (II) hence functions like a surfactant and thereby has the effect of accelerating the mixing of all polycarbonate raw materials.

Examples of the dihydroxy compound (II) include aliphatic dihydroxy compounds such as ethylene glycol, 1,3-propanediol, 1,2-propanediol, 1,4-butanediol, 1,3-butanediol, 1,2-butanediol, 1,5-heptanediol, and 1,6-hexanediol, oxyalkylene glycols such as diethylene glycol, triethylene glycol, and tetraethylene glycol, and alicyclic dihydroxy compounds such as 1,2-cyclohexanedimethanol, 1,3-cyclohexanedimethanol, 1,4-cyclohexanedimethanol, tricycledecanedimethanol, pentacyclopentadecanedimethanol, 2,6-decalindimethanol, 1,5-decalindimethanol, 2,3-decalindimethanol, 2,3-norbornanedimethanol, 2,5-norbornanedimethanol, and 1,3-adamantanedimethanol. Especially preferred of these from the standpoints of optical properties, availability, and handleability are 1,3-propanediol as an aliphatic dihydroxy compound and 1,4-cyclohexanedimethanol and/or tricyclodecanedimethanol as an alicyclic hydroxy compound.

Use of the dihydroxy compound (II) not only is effective in accelerating the mixing of polycarbonate raw materials as stated above but also can produce the effects of improving flexibility, enhancing heat resistance, improving moldability, etc. However, when the proportion of structural units derived from the dihydroxy compound (II) is too high, there are cases where the inherent optical properties decrease or heat resistance decreases. Consequently, it is preferred that the proportion of the dihydroxy compound (I) to the sum of the dihydroxy compound (I) and the dihydroxy compound (II) should be 20% by mole or higher, preferably 30% by mole or higher, especially 50% by mole or higher.

The dihydroxy compound (II) may contain the same stabilizers as those for the dihydroxy compound (I), such as a reducing agent, antioxidant, free-oxygen absorber, light absorber, antacid, pH stabilizer, and heat stabilizer, so long as these stabilizers do not lessen the effects of the invention.

In the case where the dihydroxy compound (II) contains the basic stabilizers like the dihydroxy compound (I), the content thereof is generally 0.0001-1% by weight, preferably 0.001-0.1% by weight, based on the dihydroxy compound (II).

Structural units derived from dihydroxy compounds other than the dihydroxy compound (I) and dihydroxy compound (II) (hereinafter sometimes referred to as "other dihydroxy compounds") may be used as a polycarbonate raw material so long as this does not defeat the object of the invention.

When other dihydroxy compounds are used, these dihydroxy compounds function as a compatibilizing agent. Because of this, even when the dihydroxy compound (II) is not present, the raw materials can be homogenized. However, when the proportion of structural units derived from the other dihydroxy compounds is too high, there are cases where the inherent optical properties decrease or heat resistance decreases. Consequently, it is preferred that the proportion of the dihydroxy compound (I) to the dihydroxy compound ingredient (sum of the dihydroxy compound (I), the dihydroxy compound (II), and the other dihydroxy compounds) should be 20% by mole or higher, preferably 30% by mole or higher, especially 50% by mole or higher.

Examples of the other dihydroxy compounds include aromatic bisphenols such as 2,2-bis(4-hydroxyphenyl)propane [= bisphenol A], 2,2-bis(4-hydroxy-3,5-dimethylphenyl)propane, 2,2-bis(4-hydroxy-3,5-diethylphenyl)propane, 2,2-bis(4-hydroxy(3,5-diphenyl)phenyl)propane, 2,2-bis(4-hydroxy-3,5-dibromophenyl)propane, 2,2-bis(4-hydroxyphenyl)pentane, 2,4' -dihydroxydiphenylmethane, bis(4-hydroxyphenyl)methane, bis(4-hydroxy-5-nitrophenyl)methane, 1,1-bis(4-hydroxyphenyl)-ethane, 3,3-bis(4-hydroxyphenyl)pentane, 1,1-bis(4-hydroxyphenyl)cyclohexane, bis(4-hydroxyphenyl) sulfone, 2,4'-dihydroxydiphenyl sulfone, bis(4-hydroxyphenyl) sulfide, 4,4'-dihydroxydiphenyl ether, 4,4'-dihydroxy-3,3'-dichlorodiphenyl ether, and 4,4'-dihydroxy-2,5-diethoxydiphenyl ether.

From the standpoint of obtaining especially high transparency, it is preferred that the dihydroxy compound ingredient should have a metal atom content lower than 1 ppm.

The content of metal atoms means the total amount (sum) of the metal atoms contained in the dihydroxy compounds. The sum of the amounts of elements determined by ICP emission spectroscopy is taken as the content of metal atoms. Namely, in producing a polycarbonate according to the invention, the content of metal atoms in terms of weight proportion thereof to the whole weight of the dihydroxy compound ingredient is lower than 1 ppm.

Examples of the metal atoms contained in the dihydroxy compounds described above include alkali metals such as lithium, sodium, potassium, rubidium, and cesium, alkaline earth metals such as magnesium, calcium, strontium, and barium, aluminum, chromium, manganese, iron, nickel, copper, and zinc.

When the dihydroxy compounds contain atoms of such metals in a total amount of 1 ppm or larger, there are cases where such a high metal atom content is causative of coloration of the polycarbonate to be synthesized. The total

amount of metal atoms contained in the dihydroxy compound ingredient is smaller than 1 ppm, preferably smaller than 0.5 ppm, more preferably smaller than 0.1 ppm.

Incidentally, such metal atoms, in many cases, are derived from stabilizers added in order to prevent polycarbonate raw materials including the dihydroxy compound (I) according to the invention from deteriorating in property.

As such stabilizers, reducing agents and antacids are generally used. Examples of the reducing agents include sodium borohydride and aluminum lithium hydride. Examples of the antacids include hydroxides such as sodium hydroxide, potassium hydroxide, lithium hydroxide, cesium hydroxide, calcium hydroxide, barium hydroxide, and magnesium hydroxide, carbonates such as sodium carbonate, potassium carbonate, lithium carbonate, and calcium carbonate, borates such as sodium metaborate, and phosphates such as sodium phosphate and disodium hydrogen phosphate. The alkali metals or alkaline earth metals contained in such stabilizers function also as a catalyst in polymerization reaction for polycarbonate production. Consequently, such metals remaining in a feed material may influence the polymerization reaction and are hence undesirable.

An explanation is then given on aldehyde compounds contained in the dihydroxy compound (I) according to the invention. The aldehyde compounds are contained usually as decomposition products in the dihydroxy compound (I) according to the invention. Examples of the aldehyde compounds include furfural, a furfural precursor which has not undergone dehydrating cyclization, and products of furfural decomposition. Of these, furfural is detected in a slight amount in raw materials that have not been purified by distillation and is a compound which is apt to oxidatively cause coloration. It is therefore preferred to remove furfural before polymerization reaction. The content of such aldehyde compounds is preferably lower than 0.01% by weight, especially preferably lower than 0.008% by weight. In case where the content thereof is 0.01% by weight or higher, sufficient transparency cannot be obtained due to oxidative coloration.

The amount of atoms of those metals contained in the dihydroxy compound (I) according to the invention and the amount of those aldehyde compounds contained therein can be controlled by a purification method utilizing a known technique such as, e.g., a treatment with activated carbon, treatment with an ion-exchange resin, or distillation. Aldehyde compounds such as furfural have lower boiling points than the dihydroxy compound (I) according to the invention, while metal species are lowly volatile and remain in the bottoms. It is therefore possible to simultaneously remove the aldehydes as an initial distillate and the metal species as bottoms. Purification by distillation is hence especially preferred. For the distillation, a general technique may be used, such as simple distillation, multistage distillation, or thin-film distillation. These techniques may be conducted either batchwise or continuously. Of these, simple distillation can be advantageously used as a simple technique for accomplishing the object of the invention. Simple distillation can be repeatedly conducted several times without influencing the purity and transparency of the dihydroxy compound to be obtained. Since the dihydroxy compound (I) according to the invention has a low vapor pressure at ordinary pressure, it is preferred to form an inert gas atmosphere such as argon or nitrogen and then distill the hydroxy compound (I) at a reduced pressure. From the standpoint of inhibiting thermal alteration as far as possible, it is preferred to conduct the distillation at 250°C or lower, preferably 200°C or lower, especially 180°C or lower. In this case, the distillation column has an internal pressure of generally from 0.1 kPa to 6.7 kPa.

There are cases where heavy metals, such as chromium, manganese, and iron, among those metal atoms are derived from reaction vessels (reaction tanks) and the like used for polycarbonate synthesis. There is a possibility that these heavy metals might influence the transparency of the polycarbonate even when contained in an especially small amount.

It is therefore preferred that a part coming into contact with the dihydroxy compound (I) according to the invention should be made of a molybdenum-containing alloy. A typical example of the part coming into contact with the dihydroxy compound is the reaction vessel in which the dihydroxy compound is to be reacted with a carbonic acid diester. It is also preferred that a container for storing the dihydroxy compound therein should be made of a molybdenum-containing alloy. In the case where the dihydroxy compound is distilled before being subjected to reaction, it is preferred that the distillation apparatus to be used for the distillation should be made of a molybdenum-containing alloy. It is more preferred that the part coming into contact with the dihydroxy compound having a temperature of 70°C or higher should be made of a molybdenum-containing alloy. It is even more preferred that the whole part coming into contact with the dihydroxy compound having a temperature of 70°C or higher should be made of a molybdenum-containing alloy.

The molybdenum-containing alloy may be any alloy containing molybdenum. It is, however, preferred to use a molybdenum-containing stainless steel. Stainless steels are alloy steels constituted of iron and at least 10.5% by weight chromium incorporated therein. The content of molybdenum is generally 0.1% by weight or higher, preferably 0.5% by weight or higher, more preferably 1% by weight or higher, especially preferably 1.5% by weight or higher. Meanwhile, the content thereof is generally 10% by weight or lower, preferably 5% by weight or lower, especially preferably 4% by weight or lower. In case where the value thereof is too small, the effect of yielding a polycarbonate of satisfactory quality having excellent transparency and a sufficiently high viscosity is lessened. On the other hand, even when molybdenum is contained excessively, the effect of the invention tends not to be enhanced any more. In addition, such alloys are expensive, making it difficult to produce a polycarbonate at low cost. Examples of the metals contained in the molybdenum-containing alloy include chromium and nickel besides molybdenum.

The content of chromium is preferably 10% by weight or higher, more preferably 15% by weight or higher. Meanwhile, the content thereof is preferably 30% by weight or lower, more preferably 25% by weight or lower. The content of nickel is preferably 3% by weight or higher, more preferably 8% by weight or higher. Meanwhile, the content thereof is preferably 30% by weight or lower, more preferably 20% by weight or lower.

Specific examples of the molybdenum-containing alloy include SUS316, SUS316L, SUS316N, SUS317, SUS329, and SUS436 among the stainless steels shown in JIS G 4304.

In the case of purification by distillation, it is desirable that the distillation should be conducted so as to result in at least 2% by weight (preferably at least 5% by weight) initial distillate and at least 8% by weight (preferably at least 15% by weight) bottoms, provided that the amount of the dihydroxy compound subjected to the distillation is 100% by weight. When the amount of the initial distillate resulting from the distillate is smaller than 2% by weight, there is a problem that the separation of volatile ingredients including furfural is insufficient. When the amount of the bottoms is smaller than 8% by weight, there is a problem that the separation of metal salts and metal compounds is insufficient.

Another raw material in the invention is a carbonic acid diester. The carbonic acid diester is mixed with the dihydroxy compounds described above, and the resultant mixture is used as a raw material for a polycarbonate, whereby the polycarbonate can be produced by the so-called transesterification method. Examples of the carbonic acid diester to be used in the invention generally include diesters represented by the following general formula (4).

[Chem. 16]

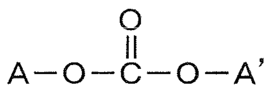

(4)

(In general formula (4), A and A' are an aliphatic group which has 1-18 carbon atoms and may have a substituent or an aromatic group which may have a substituent, provided that A and A' may be the same or different.)

Examples of the carbonic acid diesters represented by general formula (4) include diphenyl carbonate, substituted diphenyl carbonates represented by ditolyl carbonate, dimethyl carbonate, diethyl carbonate, and di-t-butyl carbonate. However, diphenyl carbonate and substituted diphenyl carbonates are preferred. Especially preferred is diphenyl carbonate. One of these carbonic acid diesters may be used alone, or a mixture of two or more thereof may be used. There are cases where carbonic acid esters contain impurities such as, e.g., chloride ions. It is therefore preferred to use a carbonic acid diester purified by, e.g., distillation.

As stated above, in the method of producing a polycarbonate of the invention, it is essential to use a dihydroxy compound ingredient at least including the dihydroxy compound (I) and a carbonic acid diester as raw materials for a polycarbonate. The dihydroxy compound (I) is mixed with the molten-state carbonic acid diester at a temperature of from 70°C to 240°C and a pressure of from 0.06 MPa to 0.20 MPa for a period of from 0.5 hours to 30 hours, and a transesterification reaction is then caused to proceed at a pressure lower than 0.06 MPa to produce a polycarbonate. From the standpoints of causing the raw-material monomer mixing and the transesterification reaction to proceed and of preventing polycarbonate from unnecessarily undergoing the transesterification reaction during the raw-material monomer mixing, use may be made of separate tanks, i.e., a tank for conducting raw-material monomer mixing at the mixing temperature and mixing pressure shown above for the mixing time shown above (hereinafter sometimes referred to as "raw-material preparation tank") and a tank for causing the transesterification reaction of the raw-material monomer mixture to proceed (hereinafter often referred to as "reaction tank"). After the dihydroxy compound ingredient is mixed with a carbonic acid diester at the mixing temperature and mixing pressure shown above for the mixing time shown above, the resultant mixture is continuously fed to a reaction tank kept at a pressure lower than 0.06 MPa to cause the transesterification reaction to proceed, whereby a polycarbonate can be produced in satisfactory yield.

It is preferred in the invention that the raw materials should be prepared so that the amount of the carbonic acid diester is 0.90-1.20 mol per mol of the dihydroxy compound ingredient to be subjected to the reaction. The molar ratio is more preferably from 0.95 to 1.10. In case where the molar ratio thereof is lower than 0.90, the polycarbonate produced has an increased amount of terminal OH groups and hence has impaired thermal stability or a desired high-molecular polymer is not obtained. In case where the molar ratio thereof is higher than 1.20, the rate of transesterification reaction decreases when the reaction is conducted under the same conditions, and a polycarbonate having a desired molecular weight is difficult to produce. In addition, the polycarbonate produced has an increased content of the residual carbonic acid diester, and this residual carbonic acid diester is a cause of odor during molding or in molded articles. Such too high molar ratios are hence undesirable.

As described above, in the method of production of the invention, the dihydroxy compound ingredient is mixed with a molten carbonic acid diester and the resultant mixture is used as a raw material for a polycarbonate.

The dihydroxy compound ingredient may be mixed in a solid state with a molten-state carbonic acid diester, or may be mixed in a molten state with a carbonic acid diester. However, from the standpoints of suitability for operation,

stable feeding, and quantitativeness, it is preferred that the dihydroxy compound ingredient in a molten state should be mixed with a carbonic acid diester.

Methods for mixing the dihydroxy compound ingredient in a molten state with a carbonic acid diester are not particularly limited. However, in the case where the dihydroxy compound ingredient includes the dihydroxy compound (II) as well as the dihydroxy compound (I), it is desirable to use a method in which the dihydroxy compound (II) in a molten state is first fed to a tank and then the dihydroxy compound (I) and a carbonic acid diester both in a molten state are fed to the tank while mixing the dihydroxy compound (I), dihydroxy compound (II), and carbonic acid diester.

Specifically, in the case of isosorbide, tricyclodecanedimethanol, and diphenyl carbonate, which are preferred typical examples of polycarbonate raw materials according to the invention, the following method may be used. Tricyclodecanedimethanol, which is an example of the dihydroxy compound (II), is fed as a first liquid feed and held at about 100°C. Subsequently, isosorbide and diphenyl carbonate, respectively as the dihydroxy compound (I) and a carbonic acid diester, are fed in a molten state, whereby all ingredients can be evenly mixed without solidifying even when an endothermic reaction occurs upon the mixing.

The raw-material preparation tank for mixing raw-material monomers in the method of production of the invention preferably is capable of heating and has a stirrer for mixing. There are no particular limitations on the shape of the tank, and vertical and horizontal ones are usable. As the stirrer, use may be made of stirrers of the high-speed rotation type, such as a turbine-stator-type high-speed rotation stirrer, disk-mill-type stirrer, and rotor-mill-type stirrer. From the standpoint of apparatus maintenance, however, a stirrer of the ordinary type constituted of a power output part, bearing, shaft, and stirring blades is preferred.

Methods of stirring are not particularly limited. Besides the ordinary method in which the liquid reaction mixture in the reaction tank is directly stirred with a stirrer disposed in an upper, lower, or lateral part of the reaction tank, use can be made of a method in which part of the liquid reaction mixture is taken out of the reaction vessel through piping or the like, stirred with a line mixer or the like, and circulated.

With respect to the kind of stirring blades, known ones can be selected. Examples thereof include anchor blades, propeller blades, screw blades, turbine blades, fan turbine blades, disk turbine blades, Pfaudler blade, full-zone blades, and Max Blend blade.

The shape of the stirring blades of the stirrer is not particularly limited. The rate of stirring is preferably from 0.05 m/s to 10 m/s, especially preferably from 0.1 m/s to 5 m/s, in terms of stirring blade tip speed. In case where the stirring blade tip speed is lower than 0.1 m/s, the effect of stirring is low and raw-material homogenization is les apt to proceed. When the stirring blade tip speed exceeds 10 m/s, power consumption by the electric motor attached to the stirring blades increases and there are cases where droplets are scattered and adhere to an upper part of the tank, etc. to form a cause of foreign matter. The term "tip speed" means the speed of the part of the stirring blades which is farthest from the stirrer shaft. By imparting pressure resistance to the raw-material preparation tank, this tank can be used also as a reaction tank for the next-step transesterification reaction.

It is preferred that the operation for mixing the dihydroxy compound ingredient with a carbonic acid diester should be conducted in an atmosphere having an oxygen concentration of 10 vol% or lower, preferably 0.0001-10 vol%, especially 0.0001-5 vol%, in particular 0.0001-1 vol%, from the standpoint of preventing deterioration.

Methods for feeding the dihydroxy compound ingredient to the tank for mixing raw-material monomers therein (raw-material preparation tank) are explained below.

In the method of production of the invention, it is preferred that the dihydroxy compound (I), which at least is included in the dihydroxy compound ingredient as a raw material, should be usually solid at ordinary temperature and have an average bulk density of generally 200-1,000 kg/$m^3$, especially 300-900 kg/$m^3$, in particular 500-900 kg/$m^3$. The term average bulk density means an average of the bulk densities of three 1-kg portions obtained by arbitrarily sampling the dihydroxy compound (I). In case where the average bulk density thereof is lower than 200 kg/$m^3$, a stable feed rate is not obtained and this results in unevenness in polymerization reaction rate or forms a cause of dust explosion. There are cases where the dihydroxy compound (I) partly becomes massive due to the influence of a slight amount of water, stabilizers, etc. contained therein or because of the method of transportation, etc., and thereby comes to have an average bulk density exceeding 1,000 kg/$m^3$. In such cases, it is preferred to crush the solid mass with a hammer mill, hammer breaker, nibbler, Fitz mill, jet mill, rubber chopper, or the like to regulate the average bulk density of the dihydroxy compound to a value within that range.

It is also preferred that the dihydroxy compound (I) should be received with a hopper having a bottom cone angle of 120 degrees or smaller, preferably 90 degrees or smaller, more preferably 70 degrees or smaller, and that the atmosphere should be regulated, while maintaining the solid state, so as to have an oxygen concentration of 10 vol% or lower, preferably 5 vol% or lower, especially preferably 2 vol% or lower, by, e.g., replacement with an inert gas, before the dihydroxy compound is brought into the state of a melt, solution, or suspension.

In the case where the dihydroxy compound (I) is melted, it is preferred that a small amount of a melt of the dihydroxy compound should be prepared beforehand and the solid-state dihydroxy compound be gradually added thereto in order to minimize thermal deterioration.

With respect to the temperature at which the dihydroxy compound (I) is melted, it is preferred to regulate the internal temperature so as to be in the range of from the melting point of the dihydroxy compound to (melting point + 50°C), especially from the melting point to (melting point + 30°C), in particular from the melting point to (melting point + 20°C), in order to minimize thermal deterioration and prevent the contents from solidifying.

The dihydroxy compound (II) can be fed by the same method as for the dihydroxy compound (I). It is desirable that the dihydroxy compound (II) should be melted by heating before being mixed. However, when the dihydroxy compound (II) is liquid at ordinary temperature, this compound may be used as it is without undergoing a heat treatment.

In the method of production of the invention, the dihydroxy compound ingredient and carbonic acid diester which have been prepared by the method described above are subjected to transesterification to produce a polycarbonate. In the method of producing a polycarbonate of the invention, basic compounds such as Group-1 metal compounds and/or Group-2 metal compounds, basic boron compounds, basic phosphorus compounds, basic ammonium compounds, and amine compounds are used alone or in combination as a polymerization catalyst (transesterification catalyst). It is, however, especially preferred to use a Group-1 metal compound and/or Group-2 metal compound alone.

Examples of the Group-1 metal compounds usable as the polymerization catalyst include sodium hydroxide, potassium hydroxide, lithium hydroxide, cesium hydroxide, sodium hydrogen carbonate, potassium hydrogen carbonate, lithium hydrogen carbonate, cesium hydrogen carbonate, sodium carbonate, potassium carbonate, lithium carbonate, cesium carbonate, sodium acetate, potassium acetate, lithium acetate, cesium acetate, sodium stearate, potassium stearate, lithium stearate, cesium stearate, sodium boron hydride, potassium boron hydride, lithium boron hydride, cesium boron hydride, phenylated sodium boride, phenylated potassium boride, phenylated lithium boride, phenylated cesium boride, sodium benzoate, potassium benzoate, lithium benzoate, cesium benzoate, disodium hydrogen phosphate, dipotassium hydrogen phosphate, dilithium hydrogen phosphate, dicesium hydrogen phosphate, disodium phenyl phosphate, dipotassium phenyl phosphate, dilithium phenyl phosphate, dicesium phenyl phosphate, alcoholates or phenolates of sodium, potassium, lithium, and cesium, and the disodium salt, dipotassium salt, dilithium salt, and dicesium salt of bisphenol A.

Examples of the Group-2 metal compounds include calcium hydroxide, barium hydroxide, magnesium hydroxide, strontium hydroxide, calcium hydrogen carbonate, barium hydrogen carbonate, magnesium hydrogen carbonate, strontium hydrogen carbonate, calcium carbonate, barium carbonate, magnesium carbonate, strontium carbonate, calcium acetate, barium acetate, magnesium acetate, strontium acetate, calcium stearate, barium stearate, magnesium stearate, and strontium stearate.

One of those metal compounds may be used alone, or two or more thereof may be used in combination.

Examples of the basic boron compounds include the sodium salts, potassium salts, lithium salts, calcium salts, barium salts, magnesium salts, or strontium salts of tetramethylboron, tetraethylboron, tetrapropylboron, tetrabutylboron, trimethylethyloron, trimethylbenzylboron, trimethylphenylboron, triethylmethylboron, triethylbenzylboron, triethylphenylboron, tributylbenzylboron, tributylphenylboron, tetraphenylboron, benzyltriphenylboron, methyltriphenylboron, and butyltriphenylboron.

Examples of the basic phosphorus compounds include triethylphosphine, tri-n-propylphosphine, triisopropylphosphine, tri-n-butylphosphine, triphenylphosphine, tributylphosphine, and quaternary phosphonium salts.

Examples of the basic ammonium compounds include tetramethylammonium hydroxide, tetraethylammonium hydroxide, tetrapropylammonium hydroxide, tetrabutylammonium hydroxide, trimethylethylammonium hydroxide, trimethylbenzylammonium hydroxide, trimethylphenylammonium hydroxide, triethylmethylammonium hydroxide, triethylbenzylammonium hydroxide, triethylpheylammonium hydroxide, tributylbenzylammonium hydroxide, tributylphenylammonium hydroxide, tetraphenylammonium hydroxide, benzyltriphenylammonium hydroxide, methyltriphenylammonium hydroxide, and butyltriphenylammonium hydroxide.

Examples of the amine compounds include 4-aminopyridine, 2-aminopyridine, N,N-dimethyl-4-aminopyridine, 4-diethylaminopyridine, 2-hydroxypyridine, 2-methoxypyridine, 4-methoxypyridine, 2-dimethylaminoimidazole, 2-methoxyimidazole, imidazole, 2-mercaptoimidazole, 2-methylimidazole, and aminoquinoline.

One of those basic compounds may be used alone, or two or more thereof may be used in combination.

The amount of the polymerization catalyst to be used is generally 0.1-100 $\mu$mol, preferably 0.5-50 $\mu$mol, per mol of the dihydroxy compound ingredient to be subjected to the reaction. In the case where a Group-1 metal compound and/or a Group-2 metal compound is employed among those compounds, this catalyst is used in an amount in the range of generally 0.1-100 $\mu$mol, preferably 0.5-50 $\mu$mol, more preferably 0.5-10 $\mu$mol, in terms of metal amount per mol of the dihydroxy compound ingredient to be subjected to the reaction. In case where the amount of the polymerization catalyst used is too small, polymerization activity necessary for producing a polycarbonate having a desired molecular weight is not obtained. On the other hand, when the polymerization catalyst is used in too large an amount, there are cases where the resultant polycarbonate has an impaired color tone and where byproducts and different kinds of bonds are formed to reduce thermal stability and flowability and cause gelation, etc., making it difficult to produce a polycarbonate of target quality.

It is preferred that the molecular weight of the polycarbonate according to the invention should be 0.40 dL/g or

higher, especially 0.45 dL/g or higher, in terms of reduced viscosity determined by using a 1/1 by weight phenol/1,1,2,2-tetrachloroethane mixed solution as a solvent to prepare a polycarbonate solution precisely regulated so as to have a polycarbonate concentration of 1.00 g/dL and measuring the viscosity of this solution at a temperature of 30.0°C±0.1°C (hereinafter simply referred to as "reduced viscosity of polycarbonate"). It is preferred that the upper limit of the reduced viscosity thereof should be 2.0 dL/g or lower, especially 1.5 dL/g or lower, in particular 1.2 dL/g or lower.

In case where the reduced viscosity of the polycarbonate is too low, this polycarbonate, when used as a molding material, tends to have low mechanical strength.

In case where the reduced viscosity thereof is too high, this polycarbonate tends to have reduced flowability during molding to reduce cycle characteristics and give molded articles which are apt to have enhanced birefringence.

A preferred embodiment of the method of production of the invention is explained below by reference to drawings. However, the invention should not be construed as being limited thereto.

The solid dihydroxy compound (I) is prepared in the same manner as described under (B) Method of Preparing Polycarbonate Raw Material. Namely, the stirring tank for melting shown in Fig. 1 may be used to melt the solid dihydroxy compound (I) in the same manner. In the case where the dihydroxy compound (II) and other dihydroxy compounds are solid, these compounds are melted using the same apparatus.

The dihydroxy compound (I) which has been melted and has been purified according to need is sent through a line (5Ax) to a storage tank (5x) equipped with a heat-medium jacked, as shown in Fig. 3. Meanwhile, the dihydroxy compound (II) which has been melted or is in a liquid state is sent through a line (7Ax) to a storage tank (7x) equipped with a heat-medium jacket, as shown in Fig. 3. On the other hand, a carbonic acid diester which has been externally fed in a molten state is sent through a line (6Ax) to a storage tank (6x) equipped with a heat-medium jacket, and is sent in a given amount through a line (6Bx) to a raw-material preparation tank (8x) equipped with a heat-medium jacket and a stirrer, while being metered with a constant delivery pump (not shown) or flow meter (not shown). The dihydroxy compound (I) and dihydroxy compound (II) both in a molten state (liquid) are sent in given amounts respectively from the storage tank (5x) and storage tank (7x) to the raw-material preparation tank (8x), while being metered with a constant delivery pump (not shown) or flow meter (not shown). The dihydroxy compounds (I) and (II) are evenly mixed with the molten carbonic acid diester with the stirrer for a period of from 0.5 hours to 30 hours while regulating the internal temperature to 70-240°C (preferably 80-120°C) with the jacket and regulating the internal pressure of the tank at 0.06-0.20 MPa. The resultant mixture is used as a raw material for a polycarbonate.

A plurality of storage tanks may be disposed to feed other dihydroxy compounds. The raw material which has been prepared is analyzed for dihydroxy compound/carbonic acid diester molar ratio according to need and then fed to a first reaction tank (9x) (see Fig. 4) through a line (9Ax).

The lines (5Ax), (5Bx), (6Ax), (6Bx), (7Ax), (7Bx), and (9Ax) are thermally insulated or heated in such a degree that the contents do not solidify, and a filter for removing foreign matter is disposed in the line (9Ax) according to need. The mixing of the molten carbonic acid diester with the dihydroxy compounds may be conducted either batchwise or continuously. The discharge of the contents (feeding to the reaction tank) may also be conducted either batchwise or continuously. However, the effects of the invention are remarkable especially in continuous discharge.

A polymerization catalyst may be added to the raw-material preparation tank (8x) or may be added to the first reaction tank (9x). However, from the standpoints of stable feeding and polymerization control, it is preferred to dispose a catalyst feed line (not shown) somewhere in the line (9Ax) and feed a catalyst therethrough. Although the raw-material preparation tank (8x) for evenly mixing raw materials has been disposed in this embodiment, this tank is not always necessary. For example, in the case where raw-material monomers are used in small amounts and are highly compatible, use may be made of a method in which the raw materials are directly fed to the first reaction tank (9x), stirred/mixed under the temperature and pressure conditions shown above, and then subjected to transesterification reaction at the pressure shown above.

In polymerizing the dihydroxy compound (I) and dihydroxy compound (II) with a carbonic acid diester by transesterification reaction in the invention, this polymerization is usually conducted in two or more steps in the presence of the catalyst described above. The mode of reaction operation may be any of a batch process, a continuous process, and a combination of batch and continuous processes.

For example, a first-step reaction is conducted at a temperature of 140-280°C, preferably 180-240°C, for 0.1-10 hours, preferably 0.5-3 hours. In the second and succeeding steps, the polymerization reaction is conducted while gradually lowering the pressure of the reaction system from the first-step pressure and simultaneously removing from the reaction system the phenol which generates. Finally, the pressure of the reaction system is regulated to 200 Pa or lower and the polymerization reaction is conducted at a temperature in the range of 210-280°C, preferably 220-260°C. From the standpoint of effective resource utilization, it is preferred that the phenol which has generated should be purified according to need and then recycled as a raw material for diphenyl carbonate, bisphenol A, etc.

From the standpoints of inhibiting the rate of polymerization from decreasing in the latter half of the polymerization reaction and of minimizing the deterioration caused by heat history, it is preferred to use a horizontal reaction tank, which is excellent in suitability for plug flow formation and interface renewal, in the final stage of the polymerization.

In this polymerization reaction, it is important to regulate a balance between temperature and the internal pressure of the reaction system. In particular, when either temperature or pressure is changed earlier than the other, there are cases where an unreacted monomer is distilled off to shift the dihydroxy compound/carbonic acid diester molar ratio, resulting in a reduced degree of polymerization. To use a reflux condenser in reactors for an initial stage, e.g., the first step, is effective in preventing that trouble.

A preferred mode of the polymerization reaction according to the invention is shown as an example in Fig. 4. Raw materials which have been mixed in the raw-material preparation tank (8x) of Fig. 3 are temporarily stocked in a raw-material storage tank (not shown) according to need and then sent through a line (9Ax) to a first reaction tank (9x), as shown in Fig. 4, preferably continuously. The first reaction tank (9x) has been connected to a vacuum pump (not shown) with a vent pipe (9Bx), and has a pressure kept at a value lower than 0.06 MPa, preferably at 0.005-0.05 MPa. Transesterification reaction proceeds in this tank (9x). The first reaction tank (9x) has an internal temperature of 150-270°C, preferably 200-240°C, and is regulated so as to result in an average residence time therein of 0.2-3 hours, preferably 0.5-2 hours. The prepolymer yielded in the first reaction tank (9x) is sent to a second reaction tank (10x) through a line (10Ax) preferably continuously. The pressure in the second reaction tank (10x) is regulated generally so as to be lower than that in the first reaction tank (9x), preferably to 1-5 kPa. The second reaction tank (10x) has an internal temperature of 200-270°C, preferably 210-250°C, and is regulated so as to result in an average residence time therein of 0.2-3 hours, preferably 0.5-2 hours. The prepolymer obtained in the second reaction tank (10x) is likewise sent through a line (11Ax) to a third reaction tank (11x), in which the polymerization reaction is caused to proceed further at a pressure generally lower than that in the second reaction tank (10x), preferably lower than 0.5 kPa, and an internal temperature of 200-270°C, preferably 210-250°C, for an average residence time of 0.2-3 hours, preferably 0.5-2 hours. The prepolymer obtained in the third polymerization tank is fed to a fourth reaction tank (12x) through a line (12Ax) with a gear pump for discharge (not shown). The fourth reaction tank (12x) is a horizontal reaction tank which is equipped with biaxially arranged stirring blades of the self-cleaning type and is constituted of a plurality of stirring-blade blocks. The prepolymer introduced into the fourth reaction tank (12x) is caused to further undergo polycondensation reaction at a pressure preferably lower than 0.3 kPa and an internal temperature of 200-280°C, preferably 210-250°C, for an average residence time of 0.2-3 hours, preferably 0.5-2 hours. Thereafter, the resultant polymer is discharged through a line (12Cx) and extruded in the form of molten strands from a die head (not shown) with a gear pump for discharge (not shown). The strands are cooled with, e.g., water and then cut with a rotary cutter (not shown) to obtain pellets. In Fig. 4, each of 9Bx, 10Bx, 11Bx, and 12Bx denotes a vent pipe, which has been connected to a vacuum pump (not shown) optionally via a condenser (not shown).

It is preferred that reaction tanks in which initial-stage reaction is conducted, e.g., the first and second reaction tanks, should be provided with a reflux condenser in order to inhibit monomer volatilization and thereby stabilize a raw-material molar ratio in the polymerization reaction.

When a polycarbonate is produced by the melt polymerization method according to the invention, a heat stabilizer can be added during or after the polymerization for the purpose of preventing coloration or thermal deterioration. In particular, it is preferred to add a heat stabilizer using a twin-screw extruder or the like after completion of the polymerization reaction.

Examples of the heat stabilizer include phosphorous acid, phosphoric acid, phosphonous acids, phosphonic acids, and esters thereof. Specific examples thereof include triphenyl phosphite, tris(nonylphenyl) phosphite, tris(2,4-di-tert-butylphenyl) phosphite, tridecyl phosphite, trioctyl phosphite, trioctadecyl phosphite, didecyl monophenyl phosphite, dioctyl monophenyl phosphite, diisopropyl monophenyl phosphite, monobutyl diphenyl phosphite, monodecyl diphenyl phosphite, monooctyl diphenyl phosphite, bis(2,6-di-tert-butyl-4-methylphenyl)penta-erythritol diphosphite, 2,2-methylenebis(4,6-di-tert-butylphenyl) octyl phosphite, bis(nonylphenyl) pentaerythritol diphosphite, bis(2,4-di-tert-butylphenyl) pentaerythritol diphosphite, distearyl pentaerythritol diphosphite, tributyl phosphate, triethyl phosphate, trimethyl phosphate, triphenyl phosphate, diphenyl mono-o-xenyl phosphate, dibutyl phosphate, dioctyl phosphate, diisopropyl phosphate, tetrakis(2,4-di-tert-butylphenyl) 4,4'-biphenylenediphosphinate, dimethyl benzenephosphonate, diethyl benzenephosphonate, and dipropyl benzenephosphonate. Preferred of these are trisnonylphenyl phosphite, trimethyl phosphate, tris(2,4-di-tert-butylphenyl) phosphite, bis(2,4-di-tert-butylphenyl) pentaerythritol diphosphite, bis(2,6-di-tert-butyl-4-methylphenyl) pentaerythritol diphosphite, and dimethyl benzenephosphonate.

As another type of heat stabilizer, use can be made of hindered phenol compounds such as, e.g., Irganox 1010 and Irganox 1076 (manufactured by Ciba-Geigy Ltd.) One of those heat stabilizers may be used alone, or two or more thereof may be used in combination. Such heat stabilizers may be additionally incorporated in addition to the amount added during the melt polymerization.

The amount of those heat stabilizers to be incorporated is preferably 0.0001-1 part by weight, more preferably 0.0005-0.5 parts by weight, even more preferably 0.001-0.2 parts by weight, per 100 parts by weight of the polycarbonate.

Furthermore, a release agent can be incorporated into the polycarbonate according to the invention in order to improve mold releasability for melt molding, so long as the incorporation thereof does not defeat the object of the invention.

Examples of the release agent include higher-fatty-acid esters of mono- or polyhydric alcohols, higher fatty acids, paraffin waxes, beeswax, olefin waxes, olefin waxes containing a carboxy group and/or carboxylic acid anhydride group, silicone oils, and organopolysiloxanes.

The higher-fatty-acid esters preferably are partial or complete esters of a mono- or polyhydric alcohol having 1-20 carbon atoms with a saturated fatty acid having 10-30 carbon atoms. Examples of the partial or complete esters of a mono- or polyhydric alcohol with a saturated fatty acid include stearic acid monoglyceride, stearic acid diglyceride, stearic acid triglyceride, stearic acid monosorbitate, stearyl stearate, behenic acid monoglyceride, behenyl behenate, pentaerythritol monostearate, pentaerythritol tetrastearate, pentaerythritol tetrapelargonate, propylene glycol monostearate, stearyl stearate, palmityl palmitate, butyl stearate, methyl laurate, isopropyl palmitate, biphenyl biphenate, sorbitan monostearate, and 2-ethylhexyl stearate.

Preferred of these are stearic acid monoglyceride, stearic acid triglyceride, pentaerythritol tetrastearate, and behenyl behenate.

The higher fatty acids preferably are saturated fatty acids having 10-30 carbon atoms. Examples of these fatty acids include myristic acid, lauric acid, palmitic acid, stearic acid, and behenic acid.

One of those release agents may be used alone, or a mixture of two or more thereof may be used.

The amount of such release agents to be incorporated is preferably 0.01-5 parts by weight per 100 parts by weight of the polycarbonate.

For incorporating various additives such as those described above into the polycarbonate according to the invention, use may be made, for example, of: a method in which the ingredients are mixed by means of a tumbling mixer, twin-cylinder mixer, supermixer, Nauta mixer, Banbury mixer, kneading rolls, extruder, or the like; or a solution blending method in which the ingredients are dissolved in a common good solvent such as, e.g., methylene chloride and mixed together in the solution state. However, methods for incorporating additives are not particularly limited, and any of the polymer blending techniques in ordinary use may be employed. Preferred of these is to mix the ingredients after completion of the polymerization by means of a single- or twin-screw extruder, in particular a twin-screw extruder, preferably while maintaining the molten state. Residual monomers and volatile matter may be removed by venting the extruder to remove the volatiles.

The temperature for melt kneading with the extruder depends on the glass transition temperature and molecular weight of the polycarbonate according to the invention. However, the temperature is generally 150-300°C, preferably 200-270°C. In case where the melt kneading temperature is lower than 150°C, the polycarbonate melt has an increased viscosity to increase the load imposed on the extruder, resulting in reduced productivity. In case where the temperature is higher than 300°C, the polycarbonate undergoes considerable thermal deterioration and this not only results in a decrease in mechanical strength due to a decrease in molecular weight but also causes coloration and gas evolution.

In the method of polycarbonate production of the invention, it is desirable to dispose a filter in order to prevent inclusion of foreign matter. The position where the filter is to be disposed is preferably on the downstream side of the extruder. The size of foreign matter particles capable of being removed by the filter (opening size) is preferably 100 $\mu$m or smaller in terms of 99% removal filtration accuracy. Especially when it is important to avoid inclusion of foreign matter, the opening size is preferably 40 $\mu$m or smaller, more preferably 10 $\mu$m or smaller.

It is desirable that extrusion of the polycarbonate in the invention should be conducted in a clean room in order to prevent inclusion of foreign matter after the extrusion.

When the polycarbonate extruded is cooled and formed into chips, it is preferred to use a method in which the extrudate is cooled with air, water, etc. It is desirable that air from which foreign matter has been removed beforehand with a high-efficiency particulate air filter or the like is used for the air cooling in order to prevent the foreign matter present in air from adhering to the extrudate again. In the case of employing water cooling, it is desirable to use water from which metallic ingredients have been removed with, e.g., an ion-exchange resin and foreign matter has been further removed with a filter. It is preferred that the filter to be used should have an opening size of 10-0.45 $\mu$m in terms of 99% removal filtration accuracy.

The polycarbonate thus obtained according to the invention or a polycarbonate composition obtained by adding various additives thereto can be molded into molded objects by a generally known technique such as, e.g., injection molding, extrusion molding, or compression molding, either directly or after having been temporarily formed into pellets with a melt extruder.

The polycarbonate produced by the method of production of the invention can be used, for example, in the field of films and sheets or in the field of lenses, e.g., camera lenses, finder lenses, and lenses for CCDs or CMOSs, or as films such as retardation films, diffusing sheets, and polarizing films for liquid-crystal or plasma displays and the like.

Furthermore, the polycarbonate according to the invention can be used also as a polymer alloy obtained by kneading the polycarbonate together with, for example, one or more of synthetic resins such as aromatic polycarbonates, aromatic polyesters, aliphatic polyesters, polyamides, polystyrene, polyolefins, acrylics, amorphous polyolefins, ABSs, and ASs, biodegradable resins such as poly(lactic acid) and poly(butylene succinate), rubbers, and the like.

EXAMPLES

The invention will be explained below in more detail by reference to Examples.

In the following, properties of polycarbonate raw materials and polycarbonates were evaluated by the following methods.

(1) Measurement of Oxygen Concentration

Oxygen concentration was measured with an oxygen analyzer (1000RS, manufactured by AMI).

(2) Determination of Sodium Concentration

Sodium concentration was determined with ICP emission spectrophotometer VISTA-PRO (manufactured by Varian Inc.).

(3) Average Bulk Density

Average bulk density was determined by measuring a volume with a measuring cylinder and calculating the density by dividing a sample weight by the volume.

(4) Water Content

Water content was determined with a Karl Fischer moisture meter (CA-200, manufactured by Mitsubishi Chemical Corp.).

(5) Color

<Method of Measuring Color (b* value)>

A color meter ("300A", manufactured by Nippon Denshoku Kogyo K.K.) was used to determine the color of chips. A given amount of chips were placed in a glass cell and examined through a reflectance measurement to determine the value of b* as provided for by the International Commission on Illumination (CIE).

The smaller the value thereof, the lower the yellowness and better the color tone.

<Method of Measuring Color (APHA)>

Color (APHA) was measured with the following apparatus.

Colorimetric color-difference meter: ZE2000 (manufactured by Nippon Denshoku Kogyo K.K.)

Isosorbide was diluted with water (sample concentration, 20% by weight), and this dilution was examined for APHA value through a transmittance measurement. The smaller the value thereof, the lower the yellowness.

(6) Reduced Viscosity

A Ubbelohde viscometer was used in automatic viscometer DT-504, manufactured by Chuo Rika Corp. A 1/1 by weight mixture of phenol and 1,1,2,2-tetrachloroethane was used as a solvent to measure the viscosity at a temperature of 30.0°C±0.1°C. A solution was precisely prepared so as to have a concentration of 1.00 g/dL.

A sample was dissolved with stirring at 120°C for 30 minutes, and the resultant solution was subjected to the measurement after cooling.

Relative viscosity ηrel was determined from the flow time for the solvent $t_0$ and the flow time for the solution t using the following equation.

$$\eta rel = t/t_0$$

Specific viscosity ηsp was determined from the relative viscosity ηrel using the following equation.

$$\eta sp = (\eta - \eta_0)/\eta_0 = \eta rel - 1$$

The specific viscosity ηsp was divided by the concentration c (g/dL) to determine the reduced viscosity ηred using the following equation.

$$\eta red = \eta sp/c$$

The larger the value thereof, the higher the molecular weight.

(7) Determination of Formic Acid

Isosorbide was diluted with pure water 100 times, and this dilution was examined with ion chromatograph Type DX-500, manufactured by Dionexy.

The isosorbide used was one manufactured by Roquette Freres; 1,4-cyclohexanedimethanol by Eastman Chemical Co.; cesium carbonate by Wako Pure Chemical Ltd.; diphenyl carbonate by Mitsubishi Chemical Corp.; tricyclodecanedimethanol by Orcea; 1,4-butanediol by Mitsubishi Chemical Corp.; and 9,9-bis(4-(2-hydroxyethoxy)phenyl)fluorene by Osaka Gas Chemical Co., Ltd.

(8) Method of Determining Purity

The purity of isosorbide was determined with the following apparatus.

Gas chromatograph: HP6890 (manufactured by Hewlet-Packard Co.)
Column: DB-1 (manufactured by J&W Scientific)

A test sample was diluted with special-grade reagent acetonitrile 20-100 times.

(9) Method of Determining Metal Atom Content

The metal atom content (ppm by weight) of isosorbide was determined with the following apparatus.

ICP emission apparatus: VISTA-PRO (manufactured by Varian Inc.)

The following are abbreviations for compounds used in the Examples given below.

ISB: isosorbide
CHDM: 1,4-cyclohexanedimethanol
TCDDM: tricyclodecanedimethanol
BD: 1,4-butanediol
BHEPF: 9,9-bis(4-(2-hydroxyethoxy)phenyl)fluorene
DPC: diphenyl carbonate

EXAMPLE 1

Isosorbide (ISB: melting point, 66°C) flakes having a bulk density of 750 kg/$m^3$ and containing 30 ppm by weight disodium hydrogen phosphate as a stabilizer were packed into a paper bag having an inner bag constituted of a 100-μm film obtained by vapor-depositing aluminum on a film made of polyethylen. The atmosphere in the paper bag was replaced with nitrogen to regulate the oxygen concentration therein to 1-2%. Thereafter, the paper bag was hermetically sealed by heat sealing. This paper bag was placed in a 10°C thermostatic chamber, and a load was imposed thereon from above so as to result in a pressure at the bottom of 0.029 kgf/$cm^2$. The packed flakes in this state were stored for 60 days, and the contents were examined thereafter. As a result, the flakes were found to retain the original state observed at the time of packing, and had not become a solid mass. The flakes at this time had a water content of 0.2% by weight and a formic acid content of 5 ppm by weight or lower. A hundred parts by weight of these ISB flakes were introduced beforehand in a nitrogen stream (oxygen concentration, 0.0005-0.001 vol%) into a vessel equipped with stirring blades, and were heated with a heat medium. At the time when the contents had begun to melt and become capable of being stirred, stirring was initiated to evenly melt the whole contents and the internal temperature was adjusted to 80°C. Subsequently, the same ISB flakes as those described above were introduced into the hopper of a weigh feeder, and the atmosphere in the hopper was sufficiently replaced with nitrogen. Thereafter, the flaky ISB was fed, at a rate of 200 parts by weight per hour, to the molten ISB present in the vessel and was continuously melted. The contents of the vessel were continuously stirred during the feeding of the ISB flakes, and the feeding was stopped at the time when the ISB flakes had been fed in a total amount of 400 parts by weight. Although a slight decrease in internal temperature was

observed during the ISB flake feeding, this temperature decrease did not result in solidification. After the stop of the feeding, the internal temperature was rapidly returned to 80°C. Subsequently, the pressure of the vessel was gradually lowered and the contents were heated. At the time when the internal pressure and the internal temperature became 133-266 Pa and 160°C, respectively, a distillate began to be obtained. An initial distillate was obtained in an amount of 25.5 parts by weight, followed by 403.5 parts by weight of a main distillate and 28.5 parts by weight of a final distillate. The remainder was left as bottoms in the vessel. This purified ISB had a formic acid concentration of 5 ppm by weight or lower and a sodium concentration of 0.1 ppm by weight or lower.

Into a polymerizer equipped with stirring blades was introduced 27.2 parts by weight of tricyclodecanedimethanol (TCDDM; viscous liquid). The atmosphere in the polymerizer was sufficiently replaced with nitrogen (oxygen concentration, 0.0005-0.001 vol%). Thereafter, 100 parts by weight of diphenyl carbonate (DPC) which had been purified by distillation to a chloride ion concentration of 10 ppb or lower was introduced into the polymerizer in a nitrogen atmosphere while keeping the DPC at 100°C in a liquid state. Subsequently, while the contents were being stirred, the mixture of the TCDDM and the DPC was homogenized and heated with a heat medium to maintain an internal temperature of 100°C. In a nitrogen stream, the main distillate of ISB which had been purified by distillation by the method described above was then continuously fed at a rate of 300 parts by weight per hour in a nitrogen atmosphere (oxygen concentration, 0.0005-0.001 vol%) while keeping the ISB at 80°C in a liquid state. The contents were continuously stirred during the feeding of the ISB, and the feeding was stopped at the time when the ISB had been fed in an amount of 47.3 parts by weight. Although a slight decrease in internal temperature was observed during the ISB feeding, the mixture was always homogeneous. After the stop of the feeding, the internal temperature was rapidly returned to 100°C. To the polycarbonate raw material thus prepared, 0.00019 parts by weight of cesium carbonate was added as a polymerization catalyst. The internal pressure of the reaction vessel was lowered from ordinary pressure to 13.3 kPa, and the temperature of the heat medium was elevated to 190°C over 1 hour to initiate polymerization reaction. The reaction system was kept in this state for 15 minutes. Thereafter, the internal pressure of the reaction vessel was lowered to 6.67 kPa and the temperature of the heat medium was elevated to 230°C over 15 minutes. Thereafter, the temperature of the heat medium was elevated to 250°C over 8 minutes and, simultaneously therewith, the internal pressure of the reaction vessel was reduced to 0.2 kPa or lower. The system was kept in this state for 120 minutes. Thereafter, the polymer obtained was discharged as strands, solidified by water cooling, and then cut to obtain pellets. The polymer obtained had a reduced viscosity of 0.58 dL/g and a b* value of 6.5. These results are shown in Table 1.

EXAMPLE 2

ISB was stored and polymerized in the same manners as in Example 1, except that the ISB was stored at a temperature of 20°C. The polymer obtained had a reduced viscosity of 0.55 dL/g and a b* value of 6.9. These results are shown in Table 1.

EXAMPLE 3

ISB was stored and polymerized in the same manners as in Example 2, except that when the ISB was stored, ten bags of an iron-based free-oxygen absorber (trade name, Ageless SA100; manufactured by Mitsubishi Gas Chemical Co., Ltd.) were caused to coexist. The polymer obtained had a reduced viscosity of 0.58 dL/g and a b* value of 6.3. These results are shown in Table 1.

EXAMPLE 4

ISB was stored and polymerized in the same manners as in Example 1, except that the ISB storage temperature was changed to 45°C. The polymer obtained had a reduced viscosity of 0.53 dL/g and a b* value of 7.4. These results are shown in Table 1.

EXAMPLE 5

ISB was stored and polymerized in the same manners as in Example 1, except that the ISB storage temperature was changed to 45°C and that a polyethylene film having a thickness of 100 μm which had not undergone aluminum vapor deposition was used as an inner bag. After the storage, the ISB had a water content of 0.3% by weight. The polymer obtained had a reduced viscosity of 0.50 dL/g and a b* value of 9.0. These results are shown in Table 1.

EXAMPLE 6

ISB was stored and polymerized in the same manners as in Example 3, except that the ISB storage temperature

was changed to 45°C. The polymer obtained had a reduced viscosity of 0.57 dL/g and a b* value of 6.6. These results are shown in Table 1.

EXAMPLE 7

ISB was stored and polymerized in the same manners as in Example 1, except that the ISB storage temperature was changed to 45°C and the load was changed to 0.232 kgf/cm$^2$. After the storage of the ISB, solid masses which were thought to have been formed by compaction were partly observed. These masses were disaggregated in a nitrogen atmosphere to bring the ISB into approximately the original flaky state. Thereafter, the ISB was melted, distilled, and polymerized in the same manners as in Example 1. The polymer obtained had a reduced viscosity of 0.52 dL/g and a b* value of 8.3. These results are shown in Table 1.

COMPARATIVE EXAMPLE 1

ISB was stored and polymerized in the same manners as in Example 5, except that the ISB storage temperature was changed to 65°C. After the storage, the ISB had a water content of 0.4% by weight. After the storage, the ISB was found to become a solid mass which was thought to have been formed by compaction or fusion bonding. The solid mass had conformed to the shape of the packing material, leaving a trace of flakes. This solid mass was disaggregated in a nitrogen atmosphere to regulate this ISB so as to have approximately the same particle diameter as the original flakes. Thereafter, this ISB was melted, distilled, and polymerized in the same manners as in Example 5. The polymer obtained had a reduced viscosity as low as 0.40 dL/g and a b* value of 12.5, showing that the polymer had an impaired color tone. These results are shown in Table 1.

COMPARATIVE EXAMPLE 2

ISB was stored and polymerized in the same manners as in Example 5, except that the ISB was stored so as to have a water content of 1.5% by weight after the storage. After the storage of the ISB, solid masses which were thought to have been formed by compaction or fusion bonding were observed. These masses were disaggregated in a nitrogen atmosphere to regulate this ISB so as to have approximately the same particle diameter as the original flakes. Thereafter, this ISB was melted, distilled, and polymerized in the same manners as in Example 5. The polymer obtained had a reduced viscosity as low as 0.40 dL/g and a b* value of 10.5, showing that the polymer had an impaired color tone. These results are shown in Table 1.

COMPARATIVE EXAMPLE 3

ISB was stored and polymerized in the same manners as in Example 5, except that the load to be imposed on the ISB was changed to 0.600 kgf/cm$^2$. After the storage of the ISB, solid masses which were thought to have been formed by compaction were observed. These masses were disaggregated in a nitrogen atmosphere to regulate this ISB so as to have approximately the same particle diameter as the original flakes. Thereafter, this ISB was melted, distilled, and polymerized in the same manners as in Example 5. The polymer obtained had a reduced viscosity as low as 0.47 dL/g and a b* value of 10.0, showing that the polymer had an impaired color tone. These results are shown in Table 1.

[Table 1]

| | | Unit | Example 1 | Example 2 | Example 3 | Example 4 | Example 5 | Example 6 | Example 7 | Comparative Example 1 | Comparative Example 2 | Comparative Example 3 |
|---|---|---|---|---|---|---|---|---|---|---|---|---|
| | Storage temperature | °C | 10 | 20 | 20 | 45 | 45 | 45 | 45 | 65 | 45 | 45 |
| | Water content after storage | wt% | 0.2 | 0.2 | 0.2 | 0.2 | 0.3 | 0.2 | 0.2 | 0.4 | 1.5 | 0.3 |
| | Formic acid content after storage | weight ppm | <5 | <5 | <5 | <5 | 8 | <5 | <5 | 12 | 9 | 8 |
| | Load in storage | $kgf/cm^2$ | 0.029 | 0.029 | 0.029 | 0.029 | 0.029 | 0.029 | 0.232 | 0.029 | 0.029 | 0.600 |
| | Inner bag | - | A | A | A | A | B | A | A | B | B | B |
| | Free-oxygen absorber | - | absent | absent | present | absent | absent | present | absent | absent | absent | absent |
| | State after 60-day storage | - | no change | no change | no change | no change | no change | no change | partly massive | massive | massive | massive |
| Poly-carbonate | Reduced viscosity | dL/g | 0.58 | 0.55 | 0.58 | 0.53 | 0.50 | 0.57 | 0.52 | 0.40 | 0.40 | 0.47 |
| | b* | - | 6.5 | 6.9 | 6.3 | 7.4 | 9.0 | 6.6 | 8.3 | 12.5 | 10.5 | 10.0 |

Inner bag A: 100-μm film obtained by vapor-depositing aluminum on polyethylene film
Inner bag B: 100-μm polyethylene film

EXAMPLE 8

A hundred parts by weight of isosorbide (ISB: melting point, 66°C) containing 30 ppm by weight disodium hydrogen phosphate as a stabilizer was introduced beforehand in a nitrogen stream (oxygen concentration, 0.0005-0.001 vol%) into a vessel equipped with stirring blades, and was heated with a heat medium. At the time when the contents had begun to melt and become capable of being stirred, stirring was initiated to evenly melt the whole contents and the internal temperature was adjusted to 80°C. Subsequently, the same solid-state ISB as that described above was introduced into the hopper of a weigh feeder, and the atmosphere in the hopper was sufficiently replaced with nitrogen. Thereafter, the solid-state ISB was fed, at a rate of 200 parts by weight per hour, to the molten ISB present in the vessel and was continuously melted. The contents of the vessel were continuously stirred during the feeding of the solid ISB, and the feeding was stopped at the time when the solid ISB had been fed in a total amount of 400 parts by weight. Although a slight decrease in internal temperature was observed during the feeding of the solid ISB, this temperature decrease did not result in solidification. After the stop of the feeding, the internal temperature was rapidly returned to 80°C. Subsequently, the pressure of the vessel was gradually lowered and the contents were heated. At the time when the internal pressure and the internal temperature became 133-266 Pa and 160°C, respectively, a distillate began to be obtained. An initial distillate was obtained in an amount of 25.5 parts by weight, followed by 403.5 parts by weight of a main distillate and 28.5 parts by weight of a final distillate. The remainder was left as bottoms in the vessel. After completion of the distillation, the pressure was recovered with nitrogen. The main distillate obtained was cooled and solidified in a nitrogen atmosphere and then powdered to obtain purified ISB having a bulk density of 750 $kg/m^3$. In a nitrogen atmosphere, purified ISB was packed into an aluminum laminate bag and this bag was heat-sealed. This purified ISB was stored. The purified ISB had a formic acid concentration of 5 ppm by weight or lower and a sodium concentration of 0.1 ppm by weight.

Into a polymerizer equipped with stirring blades was introduced 27.2 parts by weight of tricyclodecanedimethanol (TCDDM; viscous liquid). The atmosphere in the polymerizer was sufficiently replaced with nitrogen (oxygen concentration, 0.0005-0.001 vol%). Thereafter, 100 parts by weight of diphenyl carbonate (DPC) which had been purified by distillation to a chloride ion concentration of 10 ppb or lower was introduced into the polymerizer in a nitrogen atmosphere while keeping the DPC at 100°C in a liquid state. Subsequently, while the contents were being stirred, the mixture of the TCDDM and the DPC was homogenized and heated with a heat medium to maintain an internal temperature of 100°C. In a nitrogen stream, the purified solid ISB was then continuously fed to the molten DPC/TCDDM mixture at a rate of 300 parts by weight per hour with a weigh feeder to melt the purified solid ISB. The contents were kept being stirred during the feeding of the ISB, and the feeding was stopped at the time when the ISB had been fed in an amount of 47.3 parts by weight. Although a slight decrease in internal temperature was observed during the ISB feeding, this temperature decrease did not result in solidification. After the stop of the feeding, the internal temperature was rapidly returned to 100°C.

To the polycarbonate raw material prepared above, 0.00019 parts by weight of cesium carbonate was added as a polymerization catalyst. The internal pressure of the reaction vessel was lowered from ordinary pressure to 13.3 kPa, and the temperature of the heat medium was elevated to 190°C over 1 hour to initiate polymerization reaction. The reaction system was kept in this state for 15 minutes. Thereafter, the internal pressure of the reaction vessel was lowered to 6.67 kPa and the temperature of the heat medium was elevated to 230°C over 15 minutes. Thereafter, the temperature of the heat medium was elevated to 250°C over 8 minutes and, simultaneously therewith, the internal pressure of the reaction vessel was reduced to 0.2 kPa or lower. The system was kept in this state for 120 minutes. Thereafter, the polymer obtained was discharged as strands, solidified by water cooling, and then cut to obtain pellets. The polymer obtained had a reduced viscosity of 0.57 dL/g and a b* value of 7.3. These results are shown in Table 2.

EXAMPLE 9

A molten mixture of DPC and TCDDM was prepared in the same manner as in Example 8, and the internal temperature was kept at 100°C. A main distillate of ISB which had been purified by distillation in the same manner as in Example 8 was continuously fed thereto at a rate of 300 parts by weight per hour in a nitrogen atmosphere (oxygen concentration, 0.0005-0.001 vol%) while keeping the ISB at 80°C in a liquid state. The contents were continuously stirred during the feeding of the ISB, and the feeding was stopped at the time when the ISB had been fed in an amount of 47.3 parts by weight. Although a slight decrease in internal temperature was observed during the ISB feeding, the mixture was always homogeneous. After the stop of the feeding, the internal temperature was rapidly returned to 100°C. The polycarbonate raw material thus prepared was polymerized in the same manner as in Example 8. The polymer obtained had a reduced viscosity of 0.58 dL/g and a b* value of 6.3. These results are shown in Table 2.

EXAMPLE 10

A hundred parts by weight of isosorbide (ISB: melting point, 66°C) containing 30 ppm by weight disodium hydrogen phosphate as a stabilizer was introduced beforehand in a nitrogen stream (oxygen concentration, 0.0005-0.001 vol%) into a vessel equipped with stirring blades, and was heated with a heat medium. At the time when the contents had begun to melt and become capable of being stirred, stirring was initiated to evenly melt the whole contents and the internal temperature was adjusted to 80°C. Subsequently, the same solid-state ISB as that described above was introduced into the hopper of a weigh feeder, and the atmosphere in the hopper was sufficiently replaced with nitrogen. Thereafter, the solid-state ISB was fed, at a rate of 1,000 parts by weight per hour, to the molten ISB present in the vessel. The feeding was stopped at the time when the solid ISB had been fed in a total amount of 400 parts by weight. During the feeding of the solid ISB, the contents of the vessel were continuously stirred. However, in the course of the feeding, the contents solidified due to a decrease in internal temperature and became unable to be stirred. The stirring was hence stopped, and heating was continued until the internal temperature rose and the contents melted. Subsequently, the ISB was purified by distillation in the same manner as in Example 8. The purified ISB obtained by distillation was cooled/solidified, powdered, and stored in the same manners as in Example 8, and was mixed with a molten mixture of TCDDM and DPC in the same manner as in Example 1 to prepare a polycarbonate raw material. Thereafter, polymerization was conducted in the same manner as in Example 8. The polymer obtained had a reduced viscosity of 0.57 dL/g and a b* value of 8.2. These results are shown in Table 2.

EXAMPLE 11

A polycarbonate raw material was prepared in the same manner as in Example 8, except that the purified solid ISB was fed to the molten mixture of TCDDM and DPC at a rate of 1,000 parts by weight per hour. During the feeding of the purified solid ISB, the contents were continuously stirred. However, in the course of the feeding, the contents solidified due to a decrease in internal temperature and an insufficient melting rate and became unable to be stirred. The stirring was hence stopped, and heating was continued until the internal temperature rose and the contents melted. After it was ascertained that the internal temperature had reached 100°C and the contents had become homogenous, stirring was initiated. Polymerization was conducted in the same manner as in Example 8. The polymer obtained had a reduced viscosity of 0.56 dL/g and a b* value of 9.2. These results are shown in Table 2.

EXAMPLE 12

A polycarbonate raw material was prepared in the same manner as in Example 8, except that the purified solid ISB was fed to a molten mixture of TCDDM (27.0 parts by weight) and DPC (100 parts by weight) at a rate of 300 parts by weight per hour in a total amount of 26.8 parts by weight, that solid-state 9,9-bis(4-(2-hydroxyethoxy)phenyl)fluorene (BHEPF; melting point, 160-164°C; bulk density, 550 kg/$m^3$) was thereafter fed in the same manner at a rate of 200 parts by weight per hour in a total amount of 60.2 parts by weight, and that the amount of the cesium carbonate to be added was changed to 0.00134 parts by weight. The liquid mixture did not solidify throughout the operation for raw-material preparation, and the BHEPF dissolved rapidly. This raw material was used to conduct polymerization in the same manner as in Example 8. The polymer obtained had a reduced viscosity of 0.65 dL/g and a b* value of 13.0. These results are shown in Table 2.

EXAMPLE 13

The same procedure as in Example 9 was conducted, except that 20.2 parts by weight of 1,4-cyclohexanedimethanol (CHDM; melting point, 32-62°C) was used in place of the TCDDM. The polymer obtained had a reduced viscosity of 0.57 dL/g and a b* value of 8.6. These results are shown in Table 2.

EXAMPLE 14

The same procedure as in Example 9 was conducted, except that the ISB was used in an amount of 59.5 parts by weight and 8.4 parts by weight of 1,4-butanediol (BD; melting point, 16°C) was used in place of the TCDDM. The polymer obtained had a reduced viscosity of 0.53 dL/g and a b* value of 9.2. These results are shown in Table 2.

COMPARATIVE EXAMPLE 4

TCDDM, DPC which had been purified by distillation to a chloride ion concentration of 10 ppb or lower and had been solidified into a flaky form, and purified solid ISB obtained by the method used in Example 8 were introduced into

a polymerizer at ordinary temperature in the same weight amounts as in Example 8. The atmosphere in the polymerizer was sufficiently replaced with nitrogen (oxygen concentration, 0.0005-0.001 vol%). To this mixture was added cesium carbonate in the same weight amount as in Example 8. Subsequently, heating was initiated so as to result in an internal temperature of 100°C. After the contents had come to partly melt and become capable of being stirred, a melting operation was conducted with stirring. This state was maintained until the whole contents became homogeneous. Thereafter, the pressure of the reaction vessel was lowered from ordinary pressure to 13.3 kPa and the temperature of the heat medium was elevated to 190°C over 1 hour to initiate polymerization reaction. A polymer was obtained in the same manner as in Example 8.

The polymer obtained had a reduced viscosity of 0.52 dL/g, which was lower than in Example 8, and a b* value of 12.0, showing that the polymer had an impaired color tone.

These results are shown in Table 2.

COMPARATIVE EXAMPLE 5

TCDDM, DPC which had been purified by distillation to a chloride ion concentration of 10 ppb or lower and had been solidified into a flaky form, purified solid ISB obtained by the method used in Example 8, and solid-state BHEPF were introduced into a polymerizer at ordinary temperature in the same weight amounts as in Example 12. The atmosphere in the polymerizer was sufficiently replaced with nitrogen (oxygen concentration, 0.0005-0.001 vol%). To this mixture was added cesium carbonate in the same weight amount as in Example 12. Subsequently, heating was initiated so as to result in an internal temperature of 100°C. After the contents had come to partly melt and become capable of being stirred, a melting operation was conducted with stirring. This state was maintained until the whole contents became homogeneous. Thereafter, the pressure of the reaction vessel was lowered from ordinary pressure to 13.3 kPa and the temperature of the heat medium was elevated to 190°C over 1 hour to initiate polymerization reaction. A polymer was obtained in the same manner as in Example 8. The polymer obtained had a reduced viscosity of 0.60 dL/g, which was lower than in Example 12, and a b* value of 14.6, showing that the polymer had an impaired color tone as compared with the polymer of Example 12. These results are shown in Table 2.

[Table 2]

| | | Example 8 | Example 9 | Example 10 | Example 11 | Example 12 | Example 13 | Example 14 | Comparative Example 4 | Comparative Example 5 |
|---|---|---|---|---|---|---|---|---|---|---|
| Raw-material ISB melted beforehand | parts by weight | 100 | 100 | 100 | 100 | 100 | 100 | 100 | 100 | 100 |
| Rate of feeding solid-state raw-material ISB | parts by weightper hour | 200 | 200 | 1000 | 200 | 200 | 200 | 200 | 200 | 200 |
| Purified solid ISB | parts by weight | 47.3 | - | 47.3 | 47.3 | 26.8 | - | - | 47.3 | 26.8 |
| Purified molten ISB | parts by weight | - | 47.3 | - | - | - | 47.3 | 59.5 | - | - |
| TCDDM | parts by weight | 27.2 | 27.2 | 27.2 | 27.2 | 27.0 | - | - | 27.2 | 27.0 |
| BHEPF | parts by weight | - | - | - | - | 60.2 | - | - | - | 60.2 |
| CHDM | parts by weight | - | - | - | - | - | 20.2 | - | - | - |
| BD | parts by weight | - | - | - | - | - | - | 8.4 | - | - |
| Purified solid DPC | parts by weight | - | - | - | - | - | - | - | 100 | 100 |
| Purified molten DPC | parts by weight | 100 | 100 | 100 | 100 | 100 | 100 | 100 | - | - |
| Cesium carbonate | parts by weight | 0.00019 | 0.00019 | 0.00019 | 0.00019 | 0.00134 | 0.00019 | 0.00019 | 0.00019 | 0.00134 |
| Rate of feeding purified ISB | parts by weightper hour | 300 | 300 | 300 | 1000 | 300 | 300 | 300 | - | - |
| Rate of feeding solid BHEPF | parts by weightper hour | - | - | - | - | 200 | - | - | - | - |

(continued)

| | | Example 8 | Example 9 | Example 10 | Example 11 | Example 12 | Example 13 | Example 14 | Comparative Example 4 | Comparative Example 5 |
|---|---|---|---|---|---|---|---|---|---|---|
| Reduced viscosity | dL/g | 0.57 | 0.58 | 0.57 | 0.56 | 0.65 | 0.57 | 0.53 | 0.52 | 0.60 |
| b* | - | 7.3 | 6.3 | 8.2 | 9.2 | 13.0 | 8.6 | 9.2 | 12.0 | 14.6 |

EXAMPLE 15

A hundred parts by weight of isosorbide (ISB: melting point, 66°C) containing 30 ppm by weight disodium hydrogen phosphate as a stabilizer was introduced beforehand in a nitrogen stream (oxygen concentration, 0.0005-0.001 vol%) into a vessel equipped with stirring blades, and was heated with a heat medium. At the time when the contents had begun to melt and become capable of being stirred, stirring was initiated to evenly melt the whole contents and the internal temperature was adjusted to 80°C. Subsequently, the same solid-state ISB as that described above was introduced into the hopper of a weigh feeder, and the atmosphere in the hopper was sufficiently replaced with nitrogen. Thereafter, the solid-state ISB was fed, at a rate of 200 parts by weight per hour, to the molten ISB present in the vessel and was continuously melted. The contents of the vessel were continuously stirred during the feeding of the solid ISB, and the feeding was stopped at the time when the solid ISB had been fed in a total amount of 400 parts by weight. Although a slight decrease in internal temperature was observed during the feeding of the solid ISB, this temperature decrease did not result in solidification. After the stop of the feeding, the internal temperature was rapidly returned to 80°C. Subsequently, the pressure of the vessel was gradually lowered and the contents were heated. At the time when the internal pressure and the internal temperature became 133-266 Pa and 160°C, respectively, a distillate began to be obtained. An initial distillate was obtained in an amount of 25.5 parts by weight, followed by 403.5 parts by weight of a main distillate and 28.5 parts by weight of a final distillate. The remainder was left as bottoms in the vessel.

Into a raw-material preparation tank (8 in Fig. 2) equipped with stirring blades and having an internal pressure regulated to 0.10 MPa (oxygen concentration, 0.0005-0.001 vol%) was introduced, through a line (7B) in a nitrogen atmosphere, 27.2 parts by weight of tricyclodecanedimethanol (TCDDM) stored in a storage tank (7) heated at 100°C. Thereafter, 100 parts by weight of diphenyl carbonate (DPC) which had been purified by distillation to a chloride ion concentration of 10 ppb or lower was introduced, while being kept at 100°C in a liquid state, into the raw-material preparation tank (8) from a storage tank (6). Subsequently, the mixture of the TCDDM and the DPC was homogenized while stirring the contents at a stirring blade tip speed of 2.51 m/s, and the internal temperature was kept at 100°C. A main distillate of ISB which had been purified by distillation by the method described above and was stored, in a liquid state at 80°C, in a storage tank (5) in a nitrogen atmosphere (oxygen concentration, 0.0005-0.001 vol%) was then continuously fed to the raw-material preparation tank (8) at a rate of 300 parts by weight per hour. During the feeding of the ISB, the stirring was continued at a stirring blade tip speed of 2.51 m/s. The feeding was stopped at the time when the ISB had been fed in an amount of 47.3 parts by weight. Although a decrease in internal temperature smaller than 10°C was observed during the ISB feeding, the internal temperature was rapidly returned to 100°C after the stop of the feeding.

After completion of the ISB feeding, the polycarbonate raw material thus prepared was mixed for 1 hour by stirring the mixture under the same conditions as shown above. Subsequently, this polycarbonate raw material was continuously fed in a given amount to a first reaction tank (9) through a line (9A). A catalyst feed line was connected to the line (9A), and an aqueous solution of cesium carbonate was continuously added as a polymerization catalyst in an amount of 2.5 μmol in terms of cesium metal amount per mol of all dihydroxy compounds. The first reaction tank had a pressure of 13.3 kPa (0.0133 MPa) and an internal temperature kept at 220°C, and the liquid level was regulated so as to result in an average residence time of 1.0 hour. The phenol vapor which had generated as a by-product of the polymerization reaction and the monomer ingredients which had volatilized in slight amounts were discharged through a vent pipe (9B) and condensed with a condenser whose temperature was regulated with a 120°C heat medium. The condensate was returned to the first reaction tank (9), and the gas remaining uncondensed was recovered after having been condensed with a condenser which was disposed downstream in the vent pipe and the temperature of which was regulated with a 45°C heat medium. A prepolymer was continuously discharged from the first reaction tank and sent to a second reaction tank (10), third reaction tank (11), and fourth reaction tank (12) in this order. The second reaction tank (10) was regulated so as to have a pressure of 2 kPa and an internal temperature of 230°C and result in an average residence time of 1.0 hour. The phenol vapor which had generated as a by-product of the polymerization reaction and the monomer ingredients which had volatilized in slight amounts were discharged through a vent pipe (10B) and condensed with a condenser whose temperature was regulated with a 120°C heat medium. The condensate was returned to the second reaction tank (10), and the gas remaining uncondensed was recovered after having been condensed with a condenser which was disposed downstream in the vent pipe and the temperature of which was regulated with a 45°C heat medium. The third reaction tank (11) was regulated so as to have a pressure of 0.2 kPa and an internal temperature of 240°C and result in an average residence time of 1.0 hour, while the fourth reaction tank (12) was regulated so as to have a pressure of 0.2 kPa and an internal temperature of 240°C and result in an average residence time of 1.5 hours. A polymer was discharged in the form of strands from the fourth reaction tank (12), cooled and solidified with a water tank, and pelletized with a rotary cutter. The polymer obtained was sufficiently dried and then examined for reduced viscosity and b* value. This polymer had a reduced viscosity of 0.64 dL/g and a b* value of 3.3. These results are shown in Table 3.

EXAMPLE 16

A polymer was obtained in the same manner as in Example 15, except that the stirring blade tip speed during raw-material preparation was changed to 0.03 m/s. The polymer obtained had a reduced viscosity of 0.51 dL/g and a b* value of 4.0. These results are shown in Table 3.

EXAMPLE 17

A polymer was obtained in the same manner as in Example 15, except that TCDDM was omitted, the amount of the ISB to be fed to the raw-material preparation tank was changed to 66.2 parts by weight, and the temperature for raw-material preparation was changed to 120°C. The polymer obtained had a reduced viscosity of 0.55 dL/g and a b* value of 4.5. These results are shown in Table 3.

EXAMPLE 18

A polymer was obtained in the same manner as in Example 17, except that the temperature for raw-material preparation was changed to 180°C. The polymer obtained had a reduced viscosity of 0.40 dL/g and a b* value of 10.5. These results are shown in Table 3.

EXAMPLE 19

Into a raw-material preparation tank (7) was introduced 27.0 parts by weight of tricyclodecanedimethanol (TCDDM) through a line (7B) in the same manner as in Example 15. Thereafter, 100 parts by weight of diphenyl carbonate (DPC) which had been purified by distillation to a chloride ion concentration of 10 ppb or lower was introduced, while being kept at 100°C in a liquid state, into the raw-material preparation tank (8) from a storage tank (6). Subsequently, the mixture of the TCDDM and the DPC was homogenized while stirring the contents at a stirring blade tip speed of 2.51 m/s, and the internal temperature was kept at 100°C.

Solid-state 9,9-bis(4-(2-hydroxyethoxy)phenyl)fluorene (BHEPF; melting point, 160-164°C; bulk density, 550 kg/$m^3$) was then fed with a weigh feeder at a rate of 200 parts by weight per hour in a total amount of 60.2 parts by weight in a nitrogen stream while continuously stirring the contents at a stirring blade tip speed of 2.51 m/s.

Subsequently, a main distillate of ISB which had been purified by distillation in the same manner as in Example 15 and was stored, in a liquid state at 80°C, in a storage tank (5) in a nitrogen atmosphere (oxygen concentration, 0.0005-0.001 vol%) was then continuously fed to the raw-material preparation tank (8) at a rate of 300 parts by weight per hour. During the feeding of the ISB, the stirring was continued at a stirring blade tip speed of 2.51 m/s. The feeding was stopped at the time when the ISB had been fed in an amount of 26.8 parts by weight. Although a decrease in internal temperature smaller than 5°C was observed during the ISB feeding, the internal temperature was rapidly returned to 100°C after the stop of the feeding.

The polycarbonate raw material thus prepared was subjected to polymerization reaction in the same manner as in Example 15, except that the amount of cesium carbonate to be fed was changed to 17.6 μmol in terms of cesium metal amount per mol of all dihydroxy compounds. The polymer obtained had a reduced viscosity of 0.71 dL/g and a b* value of 5.5. These results are shown in Table 3.

EXAMPLE 20

Raw-material preparation and polymerization reaction were conducted in the same manners as in Example 15, except that 20.2 parts by weight of 1,4-cyclohexanedimethanol (CHDM) was used in place of the TCDDM and the amount of the ISB to be fed was changed to 47.5 parts by weight. The polymer obtained had a reduced viscosity of 0.60 dL/g and a b* value of 4.5. These results are shown in Table 3.

EXAMPLE 21

Raw-material preparation and polymerization reaction were conducted in the same manners as in Example 15, except that the ISB was used in an amount of 59.5 parts by weight and 8.4 parts by weight of 1,4-butanediol (BD) was used in place of the TCDDM. The polymer obtained had a reduced viscosity of 0.57 dL/g and a b* value of 5.0. These results are shown in Table 3.

COMPARATIVE EXAMPLE 6

Raw-material preparation and polymerization reaction were conducted in the same manners as in Example 3, except that the raw-material preparation time was changed to 0.3 hours and stirring was not conducted. As a result, polymerization reaction scarcely proceeded, and no polymer was obtained.

COMPARATIVE EXAMPLE 7

Raw-material preparation and polymerization reaction were conducted in the same manners as in Example 1, except that the pressure of the raw-material preparation tank was changed to 0.05 MPa. Although polymerization reaction proceeded slightly, the resultant polymer had a reduced viscosity lower than 0.10 dL/g and was unable to be discharged as strands.

[Table 3]

| | | | | Example 15 | Example 16 | Example 17 | Example 18 | Example 19 | Example 20 | Example 21 | Comparative Example 6 | Comparative Example 7 |
|---|---|---|---|---|---|---|---|---|---|---|---|---|
| Raw-material preparation | Monomer | ISB | parts by weight | 47.3 | 47.3 | 66.2 | 66.2 | 26.8 | 47.5 | 59.5 | 66.2 | 47.3 |
| | | TCDDM | parts by weight | 27.2 | 27.2 | - | - | 27.0 | - | - | - | 27.2 |
| | | CHDM | parts by weight | - | - | - | - | - | 20.2 | - | - | - |
| | | BD | parts by weight | - | - | - | - | - | - | 8.4 | - | - |
| | | BHEPF | parts by weight | - | - | - | - | 60.2 | - | - | - | - |
| | | DPC | parts by weight | 100 | 100 | 100 | 100 | 100 | 100 | 100 | 100 | 100 |
| | Temperature | | °C | 100 | 100 | 120 | 180 | 100 | 100 | 100 | 120 | 100 |
| | Pressure | | MPa | 0.10 | 0.10 | 0.10 | 0.10 | 0.10 | 0.10 | 0.10 | 0.10 | 0.05 |
| | Preparation time | | hour | 1.0 | 1.0 | 12.0 | 12.0 | 1.0 | 1.0 | 1.0 | 0.3 | 1.0 |
| | Stirring blade tip speed | | m/s | 2.51 | 0.03 | 2.51 | 2.51 | 2.51 | 2.51 | 2.51 | 0.00 | 2.51 |
| Polymer | Reduced viscosity | | dL/g | 0.64 | 0.51 | 0.55 | 0.40 | 0.71 | 0.60 | 0.57 | - | <0.10 |
| | b* | | - | 3.3 | 4.0 | 4.5 | 10.5 | 5.5 | 4.5 | 5.0 | - | - |

EXAMPLE 22

In a nitrogen stream, 500 g of solid isosorbide was introduced into a 1-L round-bottom flask equipped with a distilling tube, temperature measuring tube, and magnetic stirrer. A Liebig condenser and a receiver were disposed on the distillate side. In order to prevent the isosorbide to be obtained as a distillate from solidifying, 80°C hot water was passed through the Liebig condenser and the parts on the distillate side were thermally insulated. In a nitrogen stream, the contents were heated to 100°C with an oil bath to melt the isosorbide and stirring was initiated. Thereafter, pressure reduction was initiated. Before the pressure was lowered to 700 Pa, foaming (vaporization of low-boiling substances such as water, formic acid, and furfural) was observed. After stop of the foaming, the contents were gradually heated and the degree of vacuum was regulated to 400 Pa. The bath temperature was set at about 170°C. At an internal temperature of 155°C, a distillate began to be obtained. At this point of time, the column top temperature was 150.5°C. An initial distillate was obtained in an amount of 20 g. Thereafter, the receiver was replaced, and a main distillate was obtained in an amount of 400 g. At this point of time, the internal temperature was from 155°C to 158°C and the column top temperature was from 150.5°C to 151°C. Although the bottoms had colored in brown, the distillates obtained were colorless and transparent.

After completion of the distillation, the internal temperature was lowered to 100°C, and nitrogen was introduced to return the pressure to ordinary pressure. The main distillate obtained which was still in an unsolidified state was cooled, solidified, and powdered in a dry box in which the atmosphere had been replaced with dry nitrogen. The isosorbide obtained had the purity shown in Table 1. The low-boiling substances, such as water, formic acid, and furfural, contained in slight amounts in the isosorbide before the distillation were collected with a cold trap (dry ice/ethanol) disposed before the vacuum pump.

Into a reaction vessel were introduced 26.9 g (0.483 mol) of the isosorbide obtained, 15.8 g (0.211 mol) of tricyclodecanedimethanol (hereinafter referred to as "TCDDM"), 57.4 g (0.709 mol) of diphenyl carbonate (hereinafter referred to as "DPC"), and $2.14\times10^{-4}$ g ($1.73\times10^{-6}$ mol) of cesium carbonate as a catalyst. In a nitrogen atmosphere, a first step of reaction was conducted in which the temperature of the heating tank was elevated to 150°C to melt the raw materials while stirring the contents according to need. Subsequently, while lowering the pressure from ordinary pressure to 13.3 kPa and elevating the temperature of the heating tank to 190°C, the phenol which generated was discharged from the reaction vessel. In a second step, the temperature of the heating tank was elevated to 220°C and the internal pressure of the reaction vessel was lowered to 200 Pa or below. The phenol which generated was distilled off. After a given stirring torque had been reached, the reaction was terminated. The reaction product yielded was extruded in water to obtain polycarbonate pellets. The results of evaluation of the polycarbonate obtained are shown in Table 4.

EXAMPLE 23

Purification by distillation was conducted by performing the same operation as in Example 22 using the same apparatus as in Example 22, except that a Vigreux column was attached in place of the distilling tube. The isosorbide obtained had the purity shown in Table 4. The isosorbide obtained was used to conduct the same operation as in Example 22. The results of evaluation of the polycarbonate obtained are shown in Table 4.

EXAMPLE 24

A hundred grams of the isosorbide obtained by purification by distillation in Example 23 was melted at 80°C, and a metallic piece of SUS316 (15 mm × 50 mm; thickness, 2 mm) was immersed therein. Thereafter, replacement with nitrogen was conducted by performing vacuum degassing/pressure recovery with nitrogen. In a nitrogen atmosphere, the isosorbide was held at 80°C with stirring for 1 week. After the 1 week, the isosorbide was solidified by cooling and powdered in a nitrogen atmosphere and was subjected to the same polymerization as in Example 22 to obtain polycarbonate pellets. The results of evaluation of the polycarbonate obtained are shown in Table 4.

EXAMPLE 25

The same polymerization operation as in Example 22 was conducted using isosorbide which had been held with heating in the same manner as in Example 24, except that the metallic piece to be immersed was changed to SUS304. The results of evaluation of the polycarbonate obtained are shown in Table 4.

COMPARATIVE EXAMPLE 8

Using isosorbide which had not been distilled and had the purity shown in Table 4 was used to conduct the

same operation as in Example 22. The results of evaluation of the polycarbonate obtained are shown in Table 4.

COMPARATIVE EXAMPLE 9

Using isosorbide which had not been distilled and had the purity shown in Table 4 was used to conduct the same operation as in Example 22. The results of evaluation of the polycarbonate obtained are shown in Table 4.

[Table 4]

| | Isosorbide concentration (%) | Metal content (ppm) | | | | Furfural concentration (%) | Color (APHA) | Color (b*) | Fractions (%) | |
|---|---|---|---|---|---|---|---|---|---|---|
| | | Na | K | Ca | Total | | Before polymerization | After polymerization | Initial distillate | Bottoms |
| Example 22 | 99.99 | 0.53 | 0.20 | 0.08 | 0.81 | 0 | 3 | 6 | 4 | 16 |
| Example 23 | 99.99 | 0.06 | - | - | 0.06 | 0 | 2 | 3 | 3 | 10 |
| Example 24 | 99.96 | 0.06 | - | - | 0.06 | 0 | 15 | 8 | | |
| Example 25 | 99.96 | 0.06 | - | - | 0.06 | 0 | 12 | 9 | | |
| Comparative Example 8 | 99.90 | 10.00 | 0.10 | - | 10.10 | 0.01 | 9 | 15 | | |
| Comparative Example 9 | 99.96 | 4.60 | 0.40 | 2.70 | 7.70 | 0.01 | 4 | 11 | | |
| "-" indicates "below detection limit". | | | | | | | | | | |

INDUSTRIAL APPLICABILITY

According to the method of storage of the invention, a dihydroxy compound to be used as a raw material for a polycarbonate can be stored over long while inhibiting the compound from deteriorating in quality and while preventing or inhibiting the compound from becoming a fusion-bonded mass due to compaction. Because of this, the dihydroxy compound in the state of being packed in a container can not only be stored in a stock room but also be transported with a truck and/or a ship or the like, while maintaining the quality thereof.

The polycarbonate prepared by the method of preparation of the invention can stably yield a polycarbonate excellent in transparency, color tone, heat resistance, moldability, and mechanical strength and having excellent optical properties.

Furthermore, the polycarbonate produced by the method of production of method of preparation of the invention is excellent in transparency, color tone, heat resistance, moldability, and mechanical strength and has excellent optical properties. Because of this, the polycarbonate can be provided as a material for use in a wide range of fields including the field of injection molding for producing electrical and/or electronic parts, automotive parts, and the like, the field of films and sheets, the field of bottles and containers required to have heat resistance, lens applications such as camera lenses, finder lenses, and lenses for CCDs and CMOSs, films or sheets such as retardation films, diffusing sheets, and polarizing films for use in liquid-crystal or plasma displays, and other applications including optical disks, optical materials, optical parts, and binders for fixing colorants, charge-transfer agents, etc. Therefore, this polycarbonate is an industrially exceedingly promising material.

DESCRIPTION OF THE REFERENCE NUMERALS AND SIGNS

1 Receiving hopper
2 Weigh feeder
3 Stirring tank equipped with jacket
4 Discharge line
5, 6 Storage tank
7 Hopper
8 Weigh feeder
9 Raw-material preparation tank
10 Raw-material storage tank
5A, 5B, 6A, 6B, 7A, 9A, 10A Line
5x, 6x, 7x Storage tank
8x Raw-material preparation tank
9x First reaction tank
10x Second reaction tank
11x Third reaction tank
12x Fourth reaction tank
5Ax, 5Bx, 6Ax, 6Bx, 7Ax, 7Bx, 9Ax, 10Ax, 11Ax, 12Ax, 12Cx Line
9Bx, 10Bx, 11Bx, 12Bx Vent pipe

## Claims

**1.** A method of storing a dihydroxy compound for use in a polycarbonate raw material, which comprises packing a solid-state dihydroxy compound having the structure represented by the following formula (1) in the molecule into a container and storing the compound packed in the container, **characterized in that** the dihydroxy compound is stored over at least 30 days without causing compaction under such conditions that the dihydroxy compound has a water content of 1.0% by weight or lower, the container has an internal temperature of 60°C or lower, and a pressure of from 0.02 kgf/cm$^2$ to 0.5 kgf/cm$^2$ (0.002 MPa to 0.05 MPa) is applied to the bottom of the container by the load of the solid dihydroxy compound,

$$-CH_2-O- \qquad (1)$$

provided that the structure of formula (1) in which the oxygen atom is bonded to a hydrogen atom is excluded.

2. The method of storage according to claim 1, **characterized in that** the dihydroxy compound has a heterocyclic structure.

3. The method of storage according to claim 1 or 2, **characterized in that** the dihydroxy compound is represented by the following general formula (2)

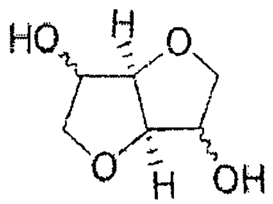

(2)

4. The method of storage according to any one of claims 1 to 3, **characterized in that** the atmosphere in the container has an oxygen concentration of from 0.0001 vol% to 10 vol%.

5. The method of storage according to any one of claims 1 to 4, **characterized in that** the container is a metallic container, a resinous container, a fiber drum, a flexible container, or a paper bag.

6. The method of storage according to claim 5, **characterized in that** the container is a container having an inner bag comprising a resinous film.

7. The method of storage according to claim 6, **characterized in that** the resinous film is a resinous film on which an inorganic layer having gas barrier properties has been formed.

8. The method of storage according to any one of claims 1 to 7, **characterized in that** the solid-state dihydroxy compound and a free-oxygen absorber are caused to coexist in the container while keeping the dihydroxy compound and the absorber not in contact with each other.

9. The method of storage according to any one of claims 1 to 8, **characterized in that** the dihydroxy compound has an average bulk density of from 200 $kg/m^3$ to 1,000 $kg/m^3$.

10. The method of storage according to any one of claims 1 to 9, **characterized in that** the dihydroxy compound has a formic acid content of 20 ppm by weight or lower.

**Patentansprüche**

1. Ein Verfahren zur Lagerung einer Dihydroxyverbindung zur Verwendung als ein Polycarbonat-Ausgangsmaterial, welches Verpacken einer Dihydroxyverbindung in festem Zustand mit der durch die folgende Formel (1) dargestellten Struktur in dem Molekül in einen Behälter und Lagerung der in dem Behälter verpackten Verbindung umfasst, **dadurch gekennzeichnet, dass** die Dihydroxyverbindung unter derartigen Bedingungen, dass die Dihydroxyverbindung einen Wassergehalt von 1,0 Gew.-% oder niedriger aufweist, der Behälter eine Innentemperatur von 60°C oder niedriger aufweist und ein Druck von 0,02 $kgf/cm^2$ bis 0,5 $kgf/cm^2$ (0,002 MPa bis 0,05 MPa) durch die Last der festen Dihydroxyverbindung auf den Boden des Behälters ausgeübt wird, über mindestens 30 Tage gelagert wird, ohne dass eine Verdichtung bewirkt wird,

$$-CH_2-O- \qquad (1)$$

mit der Maßgabe, dass die Struktur der Formel (1), in der das Sauerstoffatom an ein Wasserstoffatom gebunden ist, ausgenommen ist.

2. Das Verfahren zur Lagerung nach Anspruch 1, **dadurch gekennzeichnet, dass** die Dihydroxyverbindung eine heterocyclische Struktur aufweist.

3. Das Verfahren zur Lagerung nach Anspruch 1 oder 2, **dadurch gekennzeichnet, dass** die Dihydroxyverbindung durch die folgende allgemeine Formel (2) dargestellt ist

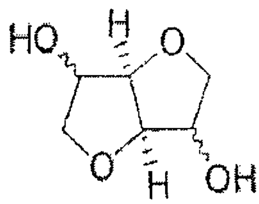

(2)

4. Das Verfahren zur Lagerung nach einem der Ansprüche 1 bis 3, **dadurch gekennzeichnet, dass** die Atmosphäre in dem Behälter eine Sauerstoffkonzentration von 0,0001 Vol.-% bis 10 Vol.-% aufweist.

5. Das Verfahren zur Lagerung nach einem der Ansprüche 1 bis 4, **dadurch gekennzeichnet, dass** der Behälter ein Metallbehälter, ein Harzbehälter, eine Fibertrommel, ein flexibler Behälter oder eine Papiertüte ist.

6. Das Verfahren zur Lagerung nach Anspruch 5, **dadurch gekennzeichnet, dass** der Behälter ein Behälter mit einem Innenbeutel, der einen Harzfilm umfasst, ist.

7. Das Verfahren zur Lagerung nach Anspruch 6, **dadurch gekennzeichnet, dass** der Harzfilm ein Harzfilm ist, auf dem eine anorganische Schicht mit Gassperreigenschaften gebildet ist.

8. Das Verfahren zur Lagerung nach einem der Ansprüche 1 bis 7, **dadurch gekennzeichnet, dass** die Dihydroxyverbindung in festem Zustand und ein Absorber für freien Sauerstoff in dem Behälter nebeneinander vorliegen, während die Dihydroxyverbindung und der Absorber nicht miteinander in Kontakt sind.

9. Das Verfahren zur Lagerung nach einem der Ansprüche 1 bis 8, **dadurch gekennzeichnet, dass** die Dihydroxyverbindung eine mittlere Schüttdichte von 200 $kg/m^3$ bis 1.000 $kg/m^3$ aufweist.

10. Das Verfahren zur Lagerung nach einem der Ansprüche 1 bis 9, **dadurch gekennzeichnet, dass** die Dihydroxyverbindung einen Ameisensäuregehalt von 20 Gewichts-ppm oder niedriger aufweist.

## Revendications

1. Méthode de stockage d'un composé dihydroxy destiné à être utilisé dans une matière première de polycarbonate, qui comprend le conditionnement d'un composé dihydroxy à l'état solide ayant la structure représentée par la formule (1) suivante dans sa molécule dans un contenant et le stockage du composé conditionné dans le contenant, **caractérisée en ce que** le composé dihydroxy est stocké pendant au moins 30 jours sans subir de tassement dans des conditions telles que le composé dihydroxy a une teneur en eau de 1,0 % en poids ou moins, le contenant a une température interne de 60 °C ou moins, et une pression de 0,02 à 0,5 $kgf/cm^2$ (0,002 à 0,05 MPa) est appliquée au fond du contenant par la charge du composé dihydroxy solide,

$$-CH_2-O- \qquad (1)$$

à condition que la structure de formule (1) dans laquelle l'atome d'oxygène est lié à un atome d'hydrogène soit exclue.

2. Méthode de stockage selon la revendication 1, **caractérisée en ce que** le composé dihydroxy a une structure hétérocyclique.

3. Méthode de stockage selon la revendication 1 ou 2, **caractérisée en ce que** le composé dihydroxy est représenté par la formule générale (2) suivante

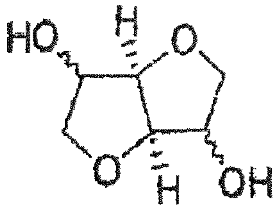

(2)

.

4. Méthode de stockage selon l'une quelconque des revendications 1 à 3, **caractérisée en ce que** l'atmosphère dans le contenant a une concentration d'oxygène de 0,0001 à 10 % en vol.

5. Méthode de stockage selon l'une quelconque des revendications 1 à 4, **caractérisée en ce que** le contenant est un contenant métallique, un contenant résineux, un fût en matière fibreuse, un contenant souple, ou un sac en papier.

6. Méthode de stockage selon la revendication 5, **caractérisée en ce que** le contenant est un contenant comportant un sac intérieur comprenant un film résineux.

7. Méthode de stockage selon la revendication 6, **caractérisée en ce que** le film résineux est un film résineux sur lequel une couche inorganique ayant des propriétés de barrière aux gaz a été formée.

8. Méthode de stockage selon l'une quelconque des revendications 1 à 7, **caractérisée en ce que** le composé dihydroxy à l'état solide et un absorbeur exempt d'oxygène libre sont amenés à coexister dans le contenant sans que le composé dihydroxy et l'absorbeur ne soient en contact l'un avec l'autre.

9. Méthode de stockage selon l'une quelconque des revendications 1 à 8, **caractérisée en ce que** le composé dihydroxy a une densité apparente moyenne de 200 à 1 000 $kg/m^3$.

10. Méthode de stockage selon l'une quelconque des revendications 1 à 9, **caractérisée en ce que** le composé dihydroxy a une teneur en acide formique de 20 ppm en poids ou moins.

[FIG. 1]

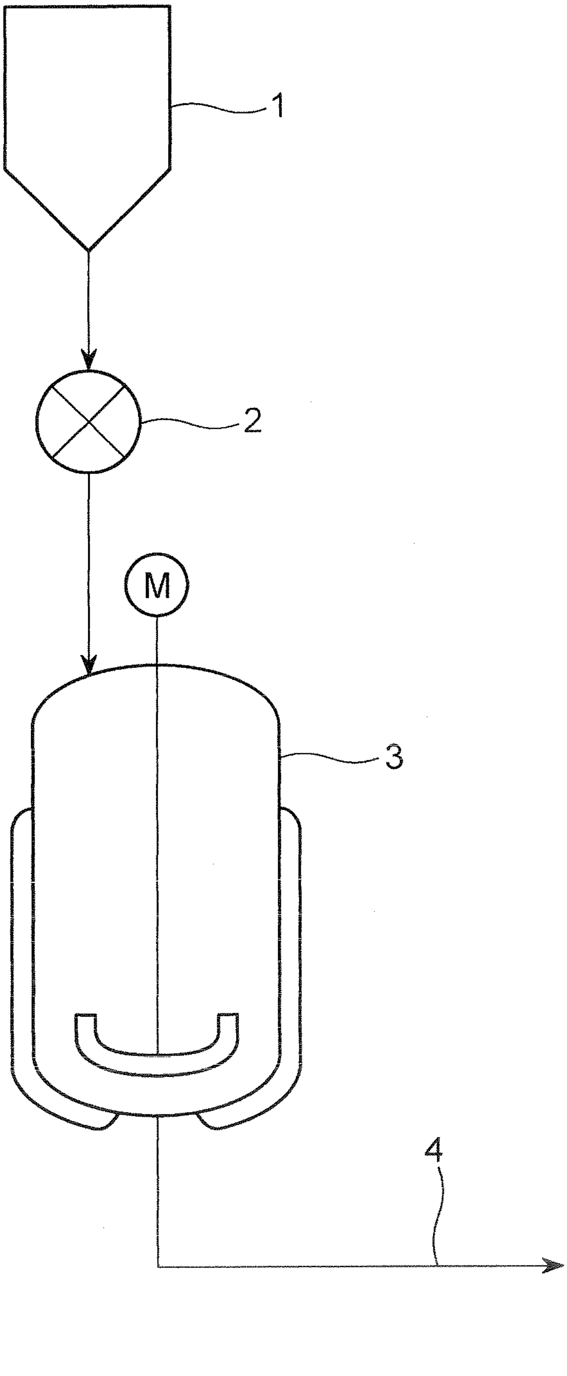

[FIG. 2]

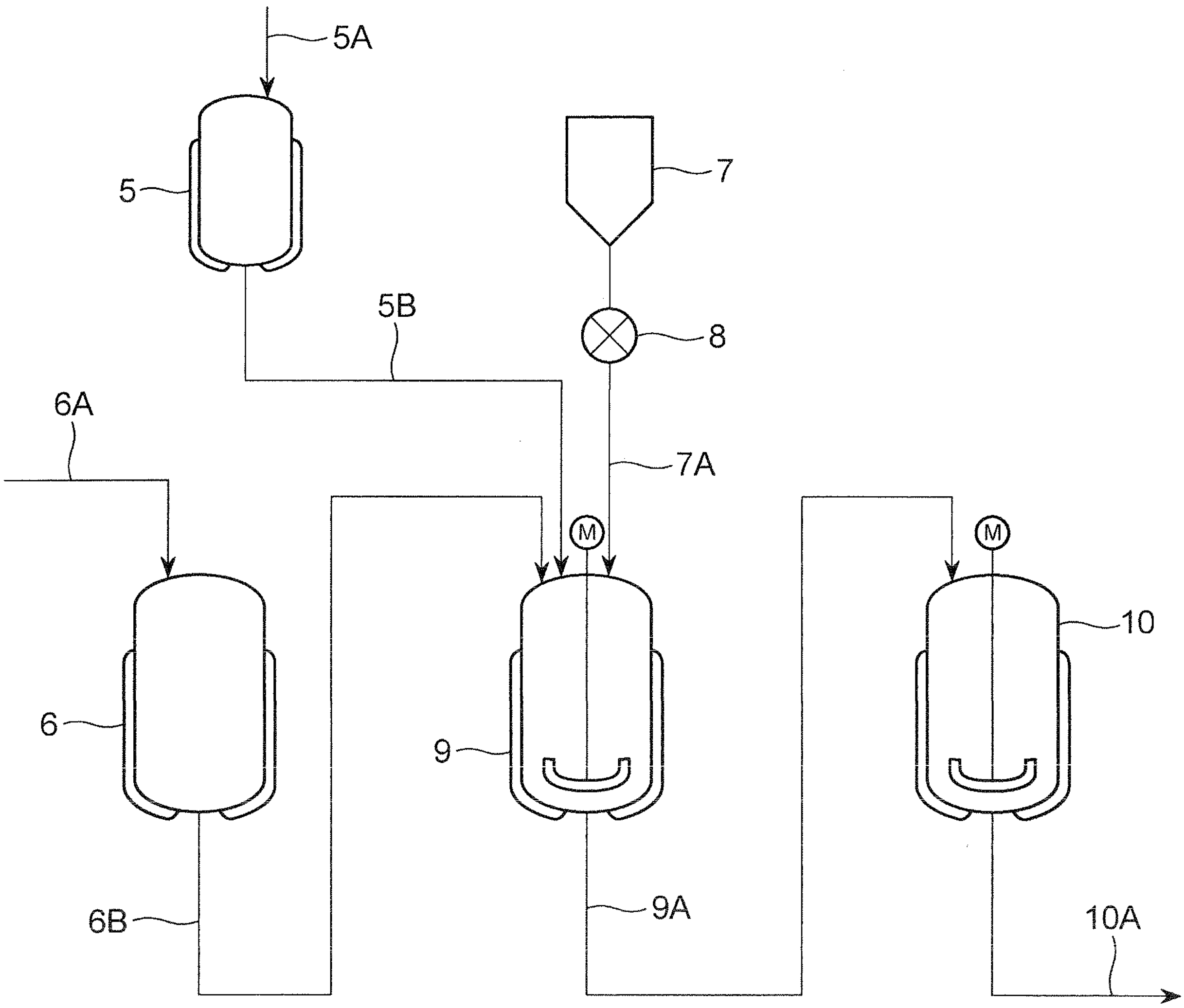

[FIG. 3]

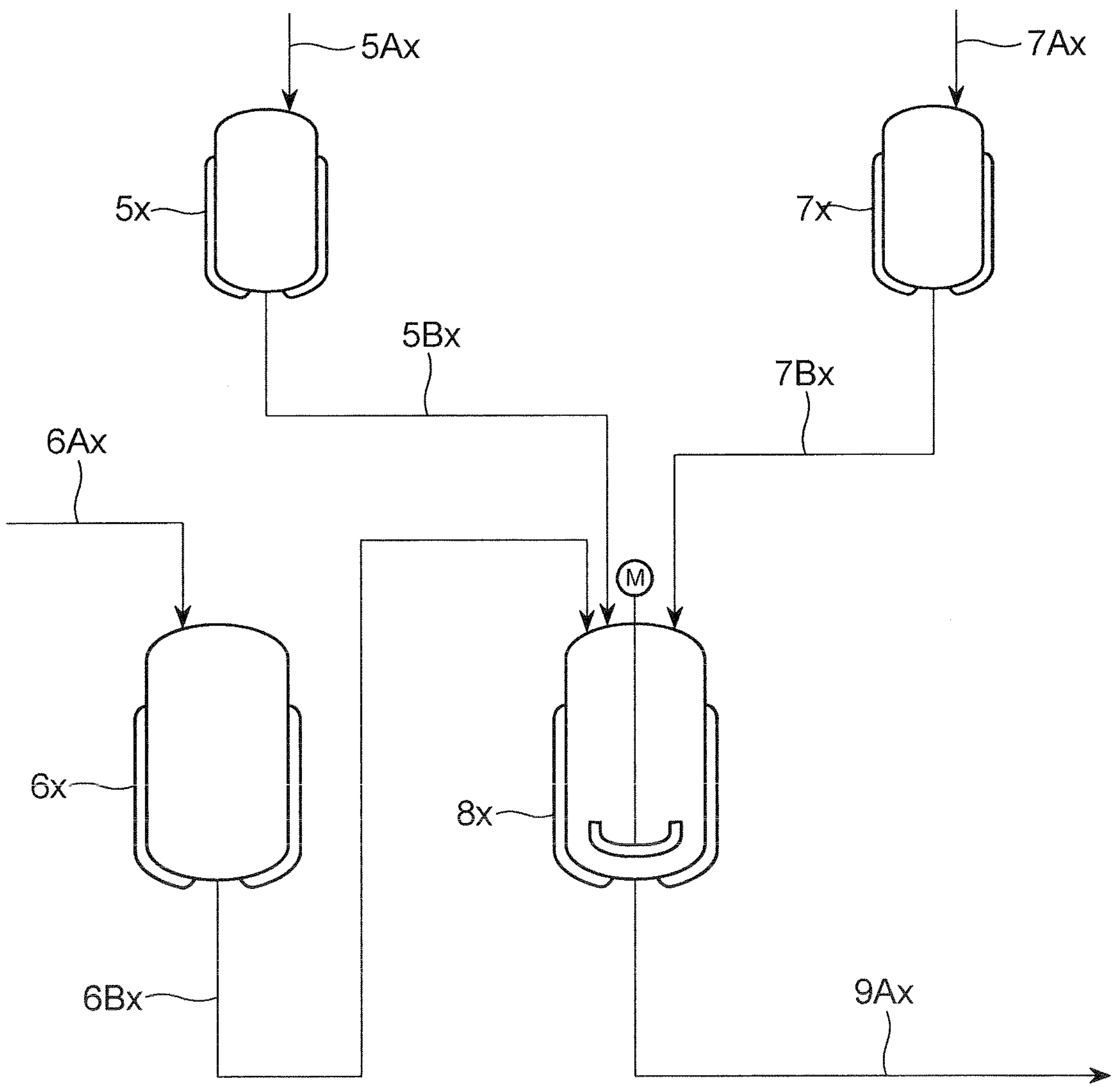

[FIG. 4]

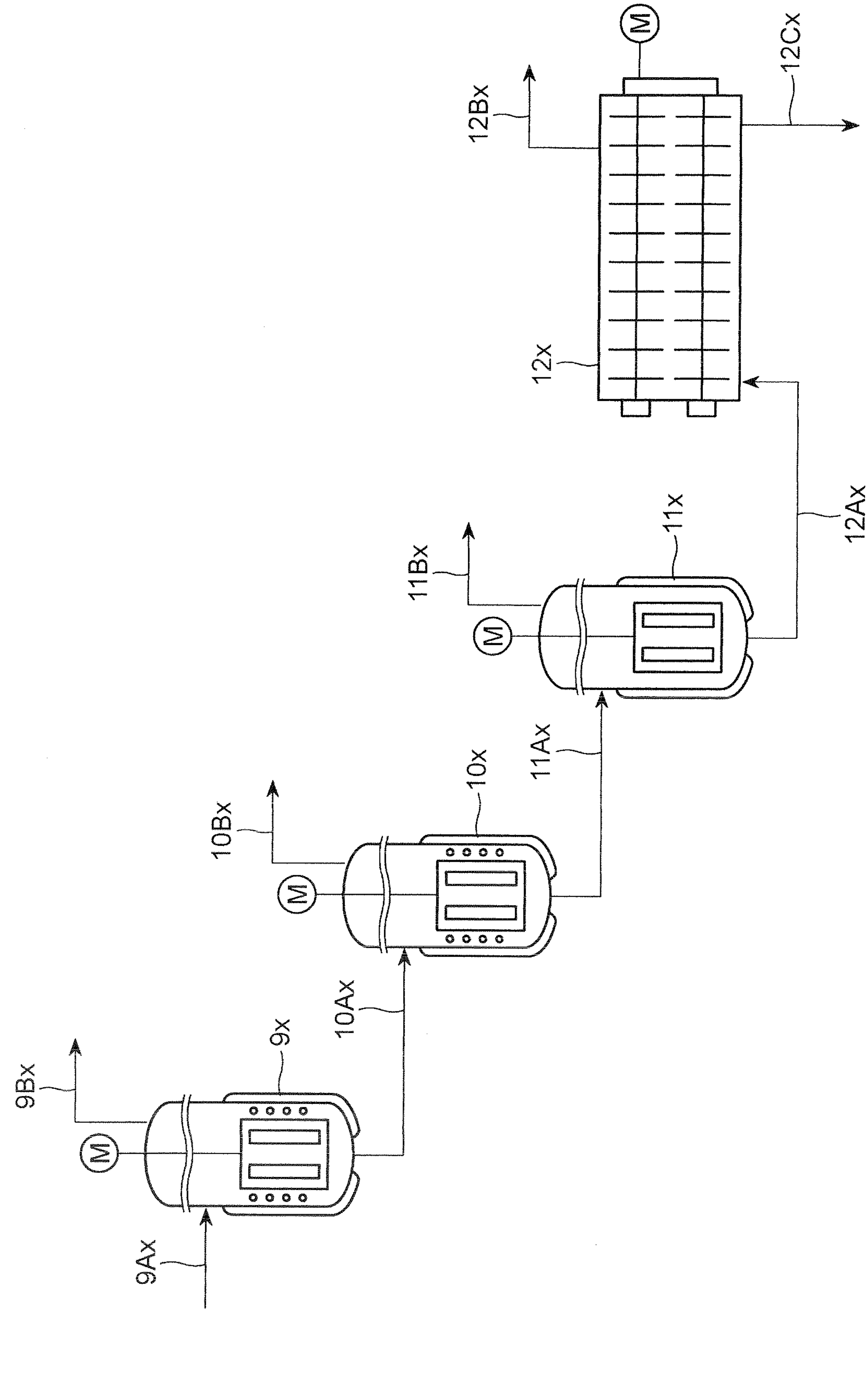

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004067990 A
- JP 2004111106 A
- JP 2006041190 A
- JP 2006232897 A
- JP 2006028441 A
- JP 2008056844 A
- JP 2008024919 A
- JP 2005509667 T
- JP 2002534486 T